(19) 

Europäisches Patentamt

European Patent Office

Office européen des brevets

(11) **EP 4 470 464 A1**

(12) **EUROPEAN PATENT APPLICATION**
published in accordance with Art. 153(4) EPC

(43) Date of publication:
04.12.2024 Bulletin 2024/49

(21) Application number: 22956093.3

(22) Date of filing: 26.08.2022

(51) International Patent Classification (IPC):
***A61B 5/22*** (2006.01)

(52) Cooperative Patent Classification (CPC):
**A61B 5/22**

(86) International application number:
**PCT/CN2022/114990**

(87) International publication number:
**WO 2024/040547 (29.02.2024 Gazette 2024/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: Shenzhen Shokz Co., Ltd.
**Shenzhen, Guangdong 518108 (CN)**

(72) Inventors:
• SU, Lei
**Shenzhen, Guangdong 518108 (CN)**
• LIU, Jia
**Shenzhen, Guangdong 518108 (CN)**
• LI, Meiqi
**Shenzhen, Guangdong 518108 (CN)**

(74) Representative: **Wang, Bo**
**Panovision IP
Ebersberger Straße 3
85570 Markt Schwaben (DE)**

(54) **SYSTEM, DEVICE, AND METHOD FOR MONITORING MOTION**

(57) One or more embodiments of the present disclosure relate to a system, a device, and a method for motion monitoring. The method includes obtaining an action signal of a user; identifying a first target interval in the action signal, the first target interval corresponding to a target action of the user; extracting, from the first target interval, a second target interval that is capable of reflecting at least one motion cycle of the target action; and evaluating the target action according to the second target interval.

<u>300</u>

```
┌─────────────────────────────────────┐
│ Obtaining an action signal of a user │──310
└─────────────────────────────────────┘
                 │
                 ▼
┌─────────────────────────────────────┐
│ Identifying a first target interval in the │──320
│ action signal, the first target interval │
│ corresponding to a target action of │
│ the user │
└─────────────────────────────────────┘
                 │
                 ▼
┌─────────────────────────────────────┐
│ Extracting, from the first target │──330
│ interval, a second target interval that │
│ is capable of reflecting at least one │
│ motion cycle of the target action │
└─────────────────────────────────────┘
                 │
                 ▼
┌─────────────────────────────────────┐
│ Evaluating the target action │──340
│ according to the second target │
│ interval │
└─────────────────────────────────────┘
```

**FIG. 3**

EP 4 470 464 A1

## Description

## TECHNICAL FIELD

The present disclosure relates to the technical field of wearable devices, and in particular, to systems, devices, and methods for motion monitoring.

## BACKGROUND

With the increasing focus on scientific exercise and physical health, devices for motion monitoring are developing significantly. Currently, the devices for motion monitoring mainly track certain physiological parameters (such as heart rate, body temperature, step frequency, blood oxygen, etc.) of users during exercise, but they are either unable to accurately monitor and provide feedback on the user's actions or need to combine multiple signals to do so. In practical scenarios, the process of monitoring and providing feedback on a user's actions often requires the involvement of professionals. For example, in a fitness scenario, users typically need the guidance of a fitness coach to continually correct their exercise movements.

Therefore, it is desirable to provide a device for motion monitoring that can guide a person to exercise, thus helping the user to exercise scientifically.

## SUMMARY

Embodiments of the present disclosure provide a method for motion monitoring. The method may include obtaining an action signal of a user; identifying a first target interval in the action signal, and the first target interval corresponding to a target action of the user; extracting, from the first target interval, a second target interval that is capable of reflecting at least one motion cycle of the target action; and evaluating the target action according to the second target interval.

In some embodiments, the action signal may include an electromyography signal or a postural signal.

In some embodiments, the identifying a first target interval in the action signal may include determining a periodic expression relating to the action signal; and determining the first target interval at least based on the periodic expression.

In some embodiments, the determining a periodic expression relating to the action signal may include obtaining an envelope signal of the action signal by smoothing the action signal; framing the envelope signal using a time window; and calculating a periodic index for each frame signal, all periodic indexes forming the periodic expression.

In some embodiments, the calculating a periodic index for each frame signal may include for each frame signal, obtaining a periodic function; determining a periodic function curve corresponding to the frame signal based on the periodic function; and determining the periodic index of the frame signal based on one or more curve features of the periodic function curve.

In some embodiments, the one or more curve features may include a peak point and a valley point of the periodic function curve.

In some embodiments, the method may further include determining a period of the frame signal based on the one or more curve features of the periodic function curve.

In some embodiments, the determining the first target interval at least based on the periodic expression may include identifying at least one candidate first target interval from the action signal based on the periodic expression according to a periodic rule; and generating the first target interval by merging or filtering the at least one candidate first target interval based on a post-processing rule.

In some embodiments, the periodic rule may include at least one of a width corresponding to continuous periodic indexes greater than a first threshold being greater than a first width threshold, a width corresponding to a distance between a first periodic index and a last periodic index that satisfy a first condition being greater than the first width threshold, wherein the first condition includes that a distance between two adjacent periodic indexes that are greater than the first threshold does not exceed a second width threshold and a difference in periods corresponding to the two adjacent periodic indexes is not greater than a first period threshold, or a sum of signal segments corresponding to periodic indexes satisfying the first condition being greater than a first length threshold.

In some embodiments, the post-processing rule may include a merge rule. The merge rule may include two adjacent candidate first target intervals satisfying that a length between an ending point of a first candidate first target interval and a starting point of a second candidate first target interval is less than a second length threshold, or a difference between the periods of two adjacent candidate first target intervals is less than a period threshold.

In some embodiments, the post-processing rule may further include a screening rule. The screening rule may include in response to determining that a periodic index corresponding to a signal after merging the two adjacent candidate first target intervals is greater than a second threshold, determining a merged interval as the first target interval; or in response to determining that the periodic index corresponding to the signal after merging the two adjacent candidate first target intervals is less than the second threshold, performing no merging.

In some embodiments, the identifying a first target interval in the action signal may include obtaining an envelope signal of the action signal by smoothing the action signal; obtaining a plurality of sets of framed signals by framing the envelope signal using a plurality of time windows of different window lengths, respectively; and calculating a periodic expression of each set of framed signals in the plurality of sets of framed signals; determining a set of transition first target intervals for each set of frame signals based on the periodic expression of each set of frame signals in the plurality of sets of frame signals ; selecting, from a plurality of sets of transition first target intervals, a set of transition first target intervals that satisfies a preference rule as a set of candidate first target intervals; and generating the first target interval by merging or filtering the set of candidate first target intervals based on the post-processing rule.

In some embodiments, the preference rule may include a sum of interval periodic indexes of the set of transition first target intervals being maximum, or the set of transition first target intervals with a more forward starting point.

In some embodiments, the determining the first target interval at least based on the periodic expression may include obtaining a trained machine learning model; and determining the first target interval by inputting the periodic expression and a period of each frame signal into the trained machine learning model.

In some embodiments, the obtaining a trained machine learning model may include obtaining a plurality of sample action signals, each of the plurality of sample action signals including a label of a sample first target interval; determining a sample periodic expression and a sample period of each sample action signal; and obtaining the trained machine learning model by training an initial machine learning model based on the sample periodic expression and the sample period of each sample action signal and the corresponding label of the sample first target interval.

In some embodiments, the identifying a first target interval in the action signal may include obtaining a trained machine learning model; and determining the first target interval by inputting the action signal into the trained machine learning model.

In some embodiments, the obtaining the trained machine learning model may include obtaining a plurality of sample action signals, each of the sample action signals including a label of a sample first target interval; and obtaining the trained machine learning model by training an initial machine learning model based on each of the plurality of sample action signals and the corresponding label of the sample first target interval.

In some embodiments, the extracting, from the first target interval, a second target interval that is capable of reflecting at least one motion cycle of the target action may include determining one or more key points in the first target interval; and determining the second target interval based on the one or more key points.

In some embodiments, the second target interval corresponds to the force exerting starting point and the force exerting ending point of the at least one motion cycle of the target action. The determining the second target interval based on the one or more key points may include centering on each of the one or more key points, taking a point with a magnitude that is a certain multiple of a maximum value of amplitude as the force exerting starting point and the force exerting ending point of the at least one motion cycle of the target action corresponding to the second target interval.

In some embodiments, the action signal may include signals from a plurality of channels, a signal of each channel corresponding to one type of force exertion muscle. Identifying a first target interval in the action signal may include determining a channel periodic expression relating to the signal of each channel. The channel periodic expression may include a periodic index of the signal of the corresponding channel; determining one or more key channels based on a plurality of channel periodic expressions relating to the signals of the plurality of channels; and determining the first target interval based on channel periodic expressions of the one or more key channels.

In some embodiments, the determining one or more key channels based on a plurality of channel periodic expressions relating to the signals of the plurality of channels may include determining a channel first target interval of each of the plurality of channels based on the plurality of channel periodic expressions; determining the one or more key channels based on an amplitude of the channel first target interval of each of the plurality of channels and a value of a periodic index corresponding to the channel first target interval.

In some embodiments, the determining the first target interval based on the channel periodic expression of the one or more key channels may include determining, based on the channel periodic expression, one or more key first target intervals of the one or more key channels, respectively; and determining the first target interval based on the one or more key first target intervals.

In some embodiments, the determining the first target interval based on the one or more key first target intervals may include determining an intersection or a union between times corresponding to the one or more key first target intervals as the first target interval, or determining an intersection or a union between times corresponding to key first target intervals that satisfy a preset condition in the one or more key first target intervals as the first target interval.

In some embodiments, the second target interval corresponds to a force exerting starting point and a force exerting ending point of the at least one motion cycle of the target action. The extracting, from the first target interval, a second target interval that is capable of reflecting at least one motion cycle of the target action may include determining one or more key points in the key first target interval of each of the one or more key channels; and determining, based on the one or more key points of each of the one or more key channels, the force exerting starting point and the force exerting ending point of the at least one motion cycle of the target action corresponding to the second target interval.

In some embodiments, the determining, based on the one or more key points of each of the one or more key channels, the force exerting starting point and the force exerting ending point of the at least one motion cycle of the target action corresponding to the second target interval may include determining, by centering on each of the one or more key points, a sub-force exerting starting point and a sub-force exerting ending point corresponding to the each of the one or more key points to obtain a candidate force segment; determining a force exertion sequence of force exertion muscles corresponding to the plurality of channels; and determining the force exerting starting point and the force exerting ending point of the at least one motion cycle of the target action corresponding to the second target interval by combining candidate force segments that satisfy the force exertion sequence.

In some embodiments, the evaluating the target action may include at least one of identifying a motion type of the target action, analyzing whether the target action is standard, determining a percentage of muscle consumption, analyzing muscle activation timing and muscle force exertion, or analyzing whether the user is fatigued.

Embodiments of the present disclosure also provide a system for motion monitoring. The system may include an acquisition module, a first target interval recognition module, a second target interval extraction module, and an evaluation module. The acquisition module may be configured to obtain an action signal of a user. The first target interval identification module may be configured to identify a first target interval in the action signal, the first target interval corresponding to a target action of the user. The second target interval recognizing module may be configured to extract, from the first target interval, a second target interval that is capable of reflecting at least one motion cycle of the target action. The evaluation module may be configured to evaluate the target action according to the second target interval.

Embodiments of the present disclosure further provide a device for motion monitoring. The device may include at least one processor and at least one memory. The at least one memory may be configured to store instructions, and the processor may be configured to execute the instructions to implement the method for motion monitoring as described above.

## BRIEF DESCRIPTION OF THE DRAWINGS

The present disclosure is further illustrated in terms of exemplary embodiments. These exemplary embodiments are described in detail with reference to according to the drawings. These embodiments are non-limiting exemplary embodiments, in which like reference numerals represent similar structures, and wherein:

FIG. 1 is a schematic diagram illustrating an application scenario of a system for motion monitoring according to some embodiments of the present disclosure;
FIG. 2 is a block diagram illustrating an exemplary system for motion monitoring according to some embodiments of the present disclosure;
FIG. 3 is a flowchart illustrating an exemplary method for motion monitoring according to some embodiments of the present disclosure;
FIG. 4 is a flowchart illustrating an exemplary process for determining a first target interval according to some embodiments of the present disclosure;
FIG. 5A is a schematic diagram illustrating an exemplary process for determining a periodic function curve of a frame signal according to some embodiments of the present disclosure;
FIG. 5B is a schematic diagram illustrating a periodic function curve of the signal 500 in FIG. 5A according to some embodiments of the present disclosure;
FIG. 6A is a schematic diagram illustrating an action signal according to some embodiments of the present disclosure;
FIG. 6B is a periodic expression of the action signal in FIG. 6A;
FIG. 7 is a flowchart illustrating an exemplary process for determining a first target interval according to some embodiments of the present disclosure;
FIG. 8 is a flowchart illustrating an exemplary process for determining a first target interval according to some embodiments of the present disclosure;
FIG. 9 is a schematic diagram illustrating an action signal including signals of a plurality of channels according to some embodiments of the present disclosure;
FIG. 10 is a flowchart illustrating an exemplary process for determining a second target interval according to some embodiments of the present disclosure; and
FIG.11 is a schematic diagram illustrating a process of determining the second target interval of the signal 900 in FIG.9.

## DETAILED DESCRIPTION

To more clearly illustrate the technical solutions related to the embodiments of the present disclosure, a brief introduction of the drawings referred to the description of the embodiments is provided below. Obviously, the drawings described below are only some examples or embodiments of the present disclosure. Those having ordinary skills in the art, without further creative efforts, may apply the present disclosure to other similar scenarios according to these drawings. Unless obviously obtained from the context or the context illustrates otherwise, the same numeral in the drawings refers to the same structure or operation.

It should be understood that "system," "device," "unit" and/or "module" as used herein is a manner used to distinguish different components, elements, parts, sections, or assemblies at different levels. However, if other words serve the same purpose, the words may be replaced by other expressions.

As shown in the present disclosure and claims, the words "one," "a," "a kind" and/or "the" are not especially singular but may include the plural unless the context expressly suggests otherwise. In general, the terms "comprise," "comprises," "comprising," "include," "includes," and/or "including," merely prompt to include operations and elements that have been clearly identified, and these operations and elements do not constitute an exclusive listing. The methods or devices may also include other operations or elements.

The flowcharts used in the present disclosure illustrate operations that systems implement according to some embodiments of the present disclosure. It should be understood that the previous or subsequent operations may not be accurately implemented in order. Instead, each step may be processed in reverse order or simultaneously. Meanwhile, other operations may also be added to these processes, or a certain step or several steps may be removed from these processes.

A system and a method for motion monitoring are provided in the present disclosure. The method may include obtaining an action signal when the user is moving and identifying a first target interval in the action signal. The first target interval corresponds to a target action of the user. Further, the method may include extracting, from the first target interval, a second target interval that is capable of reflecting at least one motion cycle of the target action and evaluating the target action according to the second target interval. According to some embodiments of the present disclosure, since most of the exercise modes are reciprocal, e.g., fitness, running, swimming, cycling, etc., their corresponding action signals (e.g., an electromyography signal, a postural signal, etc.) may show obvious periodic features. Thus, by utilizing the periodic features of the action signals (e.g., the electromyography signal), the first target interval (which may also be referred to as a periodic segment) may be identified, and further, the second target interval (i.e., a signal corresponding to the target action) may be extracted, thereby enabling identification and segmentation of the target action. Based on the identification and segmentation of the action signals, a motion type of the user, a count of actions, a quality of the action, a time of the action, or information about physiological parameters of the user when the user performs actions may be determined. In some embodiments, the method may further include generating feedback on a user's fitness action based on results of the analysis of the user's fitness action to guide the user's fitness. For example, when the user's fitness action is not standard, a prompt message (e.g., voice prompt, vibration prompt, current stimulation, etc.) may be sent to the user. The method may be applied to wearable devices (e.g., clothing, wrist guards, helmets), medical testing devices (e.g., myoelectric testers), fitness equipment, etc., and may accurately monitor and provide feedback on the user's motions by obtaining the action signals of the user, without the need for the participation of professionals, which may reduce the cost of the user's fitness while improving his or her fitness efficiency.

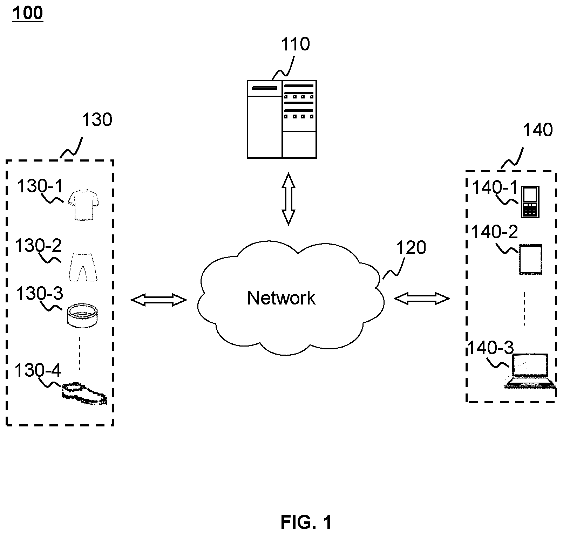

FIG. 1 is a schematic diagram illustrating an application scenario of a system for motion monitoring according to some embodiments of the present disclosure. As shown in FIG. 1, a motion monitoring system 100 may include a processing device 110, a network 120, a wearable device 130, and a terminal device 140.

The motion monitoring system 100 may obtain an action signal (e.g., an electromyography signal, a postural signal, an electrocardiographic signal, a respiratory frequency signal, etc.) used to characterize a motion action of the user and may monitor and provide feedback on the actions of the user when in motion based on the action signal of the user. For example, the motion monitoring system 100 may monitor and provide feedback on a fitness action of the user. When the user wears the wearable device 130 to perform fitness exercises, the wearable device 130 may obtain the action signal of the user. The processing device 110 or the terminal device 140 may receive and analyze the action signal of the user to determine whether the fitness action of the user is standardized. Specifically, the monitoring the action of the user may include determining a motion type, a count of actions, the quality of the action, a time of the action, or information about physiological parameters of the user when the user performs the action. Further, the motion monitoring system 100 may generate feedback on the fitness action of the user based on the result of the analysis of the fitness action of the user to guide the fitness exercises of the user. As another example, the motion monitoring system 100 may monitor and provide feedback on an action of the user while running. For example, when the user wears the wearable device 130 for running exercise, the motion monitoring system 100 may monitor whether the running action of the user is standard, whether a running time meets a health standard, etc. When the running time of the user is too long or the running action is incorrect, the wearable device may provide feedback to the user on his or her motion status to prompt the user that the user needs to adjust the running action or the running time.

The processing device 110 may be configured to process information and/or data related to the motion monitoring system 100. For example, the processing device 110 may receive the action signal (e.g., the electromyography signal) from the user and identify the first target interval in the action signal. The processing device 110 may extract, from the first target interval, the second target interval that is capable of reflecting at least one motion cycle of the user action and evaluate the action of the user based on the second target interval. In some embodiments, the processing device 110 may process data and/or information related to motion monitoring to execute program instructions related to one or more of the functions described in the present disclosure. In some embodiments, the processing device 110 may be a single server or a group of servers. The group of servers may be centralized or distributed. In some embodiments, the processing device 110 may be local or remote. In some embodiments, the processing device 110 may include one or more processing units (e.g., a single-core processing engine or a multi-core processing engine). Merely by way of example, the processing device 110 may include a central processing unit (CPU), an application-specific integrated circuit (ASIC), an application-specific instruction set processor (ASIP), a graphics processing unit (GPU), and the like. In some embodiments, the processing device 110 may be implemented on a cloud platform. For example, the cloud platform may include one or a combination of one or more of a private cloud, a public cloud, a hybrid cloud, a community cloud, a distributed cloud, a trans-cloud, a multi-cloud, and the like. In some embodiments, the processing device 110 may be a part of the wearable device 130 or the terminal device 140.

The network 120 may facilitate the exchange of data and/or information between components in the motion monitoring system 100 and/or with external devices. In some embodiments, the network 120 may be any type of wired or wireless network. Merely by way of example, the network 120 may include a cable network, a wired network (e.g., a controller area network (CAN) bus, inter-integrated circuit (I2C) bus, serial peripheral interface (SPI) communication, etc.), a fiber optic network, a telecommunications network, an intranet network, an inter-network network, a local area network (LAN), a wide area network (WAN), a wireless area network (WLAN), a metropolitan area network (MAN), a public switched telephone network (PSTN), a Bluetooth network, ZigBee network, near field communication (NFC) network, etc. or any combination thereof.

The wearable device 130 refers to a garment or device that has a wearable function. In some embodiments, the wearable device 130 may include, but is not limited to, a top device 130-1, a pants device 130-2, a wrist guard device 130-3, shoes 130-4, or the like. In some embodiments, the wearable device 130 may include a plurality of sensors. The sensors may obtain various action signals (e.g., the electromyography signal, the postural signal, temperature information, a heart rate, the electrocardiographic signal, etc.) from the user's motion. In some embodiments, the sensors may include but are not limited to one or more of an electromyography sensor, a postural sensor, a temperature sensor, a humidity sensor, an electrocardiographic sensor, an oximetry sensor, a Hall sensor, a surface electromyograph sensor, a rotation sensor, or the like. For example, an electromyography sensor may be provided at a location corresponding to a human muscle (e.g., biceps, triceps, latissimus dorsi, trapezius, etc.) in the top device 130-1, and the electromyography sensor may be fitted to the user's skin and pick up the electromyography signal when the user moves. As another example, the electrocardiographic sensor may be provided at a location in the top device 130-1 corresponding to a location near the left pectoral muscle of the human body, and the electrocardiographic sensor may capture the electrocardiographic signal of the user. As another example, the postural sensor may be provided at a location in the pants device 130-2 corresponding to a human muscle location (e.g., gluteus maximus, lateral femoral, medial femoral, gastrocnemius, etc.), and the postural sensor may collect the postural signal from the user. In some embodiments, the wearable device 130 may also provide feedback on the action of the user. For example, if a part of the user's body does not move in a standard way during exercise, the electromyography sensor corresponding to that part of the body may generate a stimulus signal (e.g., an electrical current stimulus or a strike signal) to alert the user.

It should be noted that the wearable device 130 is not limited to the top device 130-1, the pants device 130-2, the wrist guard device 130-3, and the shoes 130-4 as shown in FIG. 1, but may also include other devices applied to other devices that are required to performing motion monitoring, such as a helmet device, a knee brace device, or the like, and without limitation herein, any device that may use method for motion monitoring included in the present disclosure is within the scope of protection of the present disclosure.

The terminal device 140 may include one or more terminal devices or software. In some embodiments, the terminal device 140 may include a cell phone 140-1, a tablet 140-2, a laptop 140-3, or the like. In some embodiments, the terminal device 140 may be configured to input and/or output information and/or instructions. For example, the terminal device 140 may be connected to the wearable device 130 via the network 120, and the user may obtain a record of action of the user during exercise via the terminal device 140. The record of action may be transmitted to the processing device 110 via the terminal device 140. The processing device 110 may process the record of the action and send feedback to the terminal device 140 based on processed motion data. Exemplary feedback manners may include, but are not limited to, voice prompts, image prompts, video presentations, text prompts, or the like.

In some embodiments, the motion monitoring system 100 may further include a database. The database may be communicated over the network 120 to communicate with one or more components of the motion monitoring system 100 (e.g., the processing device 110, and the terminal device 140). In some embodiments, the database may store data and/or instructions for execution or use by the processing device 110 to perform the exemplary method described in the present disclosure.

It should be noted that the motion monitoring system 100 is provided for illustrative purposes only and is not intended to limit the scope of the present disclosure. For a person of ordinary skill in the art, a variety of modifications or variations may be made in accordance with the description in the present disclosure. For example, application scenarios may be implemented on other devices to achieve similar or different functionality. However, changes and modifications do not depart from the scope of the present disclosure.

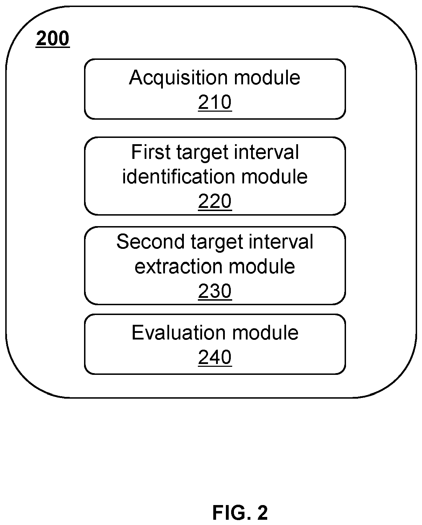

FIG. 2 is a block diagram illustrating an exemplary system for motion monitoring according to some embodiments of the present disclosure. As shown in FIG. 2, a motion monitoring system 200 may include an acquisition module 210, a first target interval identification module 220, a second target interval extraction module 230, and an evaluation module 240. In some embodiments, the motion monitoring system 200 may be part of the processing device 110 or be implemented by the processing device 110.

The acquisition module 210 may be configured to obtain an action signal of the user. In some embodiments, the action signal may include but is not limited to one or more of an electromyography signal, a postural signal, a blood pressure signal, an electrocardiographic signal, a temperature signal, a humidity signal, a blood oxygen concentration, a respiratory rate, or the like.

The first target interval identification module 220 may be configured to identify a first target interval in the action signal. The first target interval may correspond to a target action of the user. In some embodiments, the first target interval may correspond to a time period during which the user continues to perform the target action. In some embodiments, the first target interval identification module 220 may determine the first target interval based on a periodic expression relating to the action signal. In some embodiments, the first target interval identification module 220 may determine the first target interval using a trained machine learning model.

The second target interval extraction module 230 may be configured to extract, from the first target interval, a second target interval that is capable of reflecting at least one motion cycle of the target action. In some embodiments, the second target interval extraction module 230 may determine the second target interval based on one or more key points in the first target interval. In some embodiments, the second target interval may correspond to a time period during which the user completes the target action for a motion cycle.

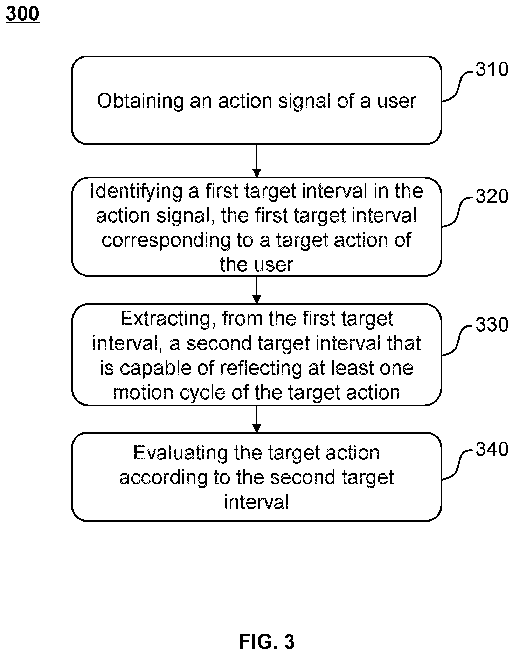

The evaluation module 240 may be configured to evaluate the target action based on the second target interval. In some embodiments, the process of evaluating the target action includes one or more of identifying a motion type of the target action, analyzing whether the target action is standard, determining muscle consumption, analyzing whether the user is fatigued, or the like. More descriptions regarding evaluating the target action based on the action signal of the user may be found elsewhere in the present disclosure (e.g., FIG. 3-FIG. 11 and their descriptions).

It should be noted that the above description of the system and its modules is provided only for descriptive convenience, and does not limit the present disclosure to the scope of the cited embodiments. It is to be understood that for a person skilled in the art, after understanding the principle of the system, it may be possible to arbitrarily combine individual modules or form a subsystem to be connected to other modules without departing from this principle. For example, the first target interval identification module 220 and the second target interval extraction module 230 may be integrated in a single component. As another example, the individual modules may share a common storage device, and the individual modules may each have their own storage device. Such morphs are within the scope of protection of the present disclosure.

FIG. 3 is a flowchart illustrating an exemplary method for motion monitoring according to some embodiments of the present disclosure. In some embodiments, process 300 may be executed by processing logic that may include hardware (e.g., circuitry, specialized logic, programmable logic, microcode, etc.), software (running on a processing device to execute instructions simulated by the hardware), etc., or any combination thereof. One or more of the operations shown in FIG.3 for motion monitoring may be realized by the motion monitoring system 100 shown in FIG.1 or the motion monitoring system 200 shown in FIG.2. For example, the process 300 may be stored in a database in the form of instructions and invoked and/or executed by the processing device 110. In some embodiments, the process 300 may include the operations described below, as shown in FIG. 3.

In 310, the processing device 110 may obtain an action signal of a user. In some embodiments, operation 310 may be performed by the acquisition module 210.

The action signal refers to information about body parameters of the user when in motion. In some embodiments, the action signal may include but is not limited to one or more of an electromyography signal, a postural signal, a blood pressure signal, an electrocardiographic signal, a temperature signal, a humidity signal, a blood oxygen concentration, a respiratory rate, or the like. For the convenience of description, the electromyography signal may be used as an example of the action signal in the present disclosure. It is to be understood that the following descriptions directed to the characteristic of the electromyography signal do not limit the scope of the present disclosure. For example, when the action signal is the postural signal, the sensor for obtaining the postural signal may include an inertial sensor (e.g., an angular velocity 3-axis sensor, an acceleration 3-axis sensor), a tensile force sensor, a stress sensor, a strain sensor, an optical sensor, or the like. For those skilled in the art, various corrections and changes may be made to the process 300 under the guidance of the present disclosure to accommodate the processing of the postural signal.

In some embodiments, the processing device 110 may obtain the action signal of the user (e.g., an electromyography signal) directly from the wearable device 130. Specifically, the user may put on or wear the wearable device 130 (e.g., an electromyography suit) and exercise. The wearable device 130 may obtain (e.g., obtain in a certain period) the action signal of the user in real-time or periodically, and transmit the acquired action signal in real-time or periodically to the processing device 110 for processing. For example, the processing device 110 may control one or more signal acquisition sensors in the wearable device 130 (e.g., all electromyography sensors on the electromyography suit) to obtain the action signal and transmit the acquired action signal to the processing device 110. In some embodiments, before starting the motion, the user may enter a name for his or her upcoming motion via the terminal device 140. The processing device 110 may control the wearable device 130 to obtain signals from one or more channels corresponding to that motion. For example, when the user performs a seated chest press, the processing device 110 may control electromyography sensors in the wearable device 130 that correspond to locations of the human pectoral muscles, latissimus dorsi muscles, or the like, to obtain the electromyography signals of corresponding muscle locations of the user. The processing device 110 may communicate with the wearable device 130 via the network 120 to obtain the obtained electromyography signals. As another example, when the user performs a deep squatting action, the processing device 110 may control electromyography sensors in the wearable device 130 that correspond to the locations of the human gluteus maximus, quadriceps, or the like, to obtain the electromyography signals of corresponding muscle locations of the user. The processing device 110 may communicate with the wearable device 130 via the network 120 to obtain the acquired electromyography signals. In some embodiments, the processing device 110 may retrieve the action signal of the user (e.g., a historical action signal of the user) from a database. Specifically, the wearable device 130 may transmit the obtained action signal of the user when in motion to the database for storage. The processing device 110 may obtain the action signal of the user from the database via the network 120.

In some embodiments, the action signal may be an action signal (e.g., the electromyography signal or the postural signal) of a particular body part of the user (e.g., arm, abdomen, etc.). The action signal of a specific body part may reflect an action status (e.g., a type of action, a magnitude of action, a frequency of action, etc., of the action of the user) of that body part when the user's body is in motion. For example, the electromyography signal of a particular body part may reflect a muscle status of that body part of the user when in motion. The electromyography signal of the body part may be used to assess the force exertion of the body part, thus evaluating whether the action of the body part is standard.

In some embodiments, the action signal may include signals from one or more channels. A signal of each channel may correspond to one type of force exertion muscle. For example, when a left-handed of the user performs a dumbbell curl action, the processing device 110 may control an electromyography sensor corresponding to the biceps muscle of the left hand to collect the electromyography signal. At this time, the action signal includes a signal from only one channel, namely, the electromyography signal of the biceps muscle of the left hand. As another example, when the user is running, the processing device 110 may control electromyography sensors corresponding to the quadriceps femoris muscle, biceps femoris muscle, gastrocnemius muscle, and gluteus maximus muscle on the left and right sides, respectively. At this time, the action signal includes signals from eight channels, which are electromyography signals of the quadriceps femoris muscle, electromyography signals of the biceps femoris muscle, electromyography signals of the gastrocnemius muscle, and electromyography signals of the gluteus maximus muscle, respectively, on the left and right sides. As another example, when the user begins to exercise, the processing device 110 may control all the electromyography sensors in the wearable device 130 to begin acquiring the electromyography signal from each muscle.

In some embodiments, the processing device 110 may obtain an envelope signal by smoothing the action signal. More description regarding the smoothing process may be found in operation 410 and related descriptions thereof. In some embodiments, the processing device 110 may sample the action signal (or the envelope signal) at a preset frequency (e.g., 100 Hz, 1000 Hz, etc.), thereby converting the action signal to a digital signal.

In 320, the processing device 110 may identify a first target interval in the action signal. The first target interval may correspond to the target action of the user. In some embodiments, operation 320 may be performed by the first target interval identification module 220.

In the present disclosure, the target action refers to an action to be monitored during the motion of the user. For example, if monitoring (e.g., evaluating) a user's running motion, the target action may be a running action. As another example, if the user is monitored for doing push-ups, the target action may be a push-up action.

The first target interval (which may also be referred to as an action first target interval) refers to a signal in the action signal that corresponds to a time period when the user does the target action. For example, during a process of running, if the user is running (i.e., rapidly alternating left and right leg steps forward) during a first time period, rests (e.g., stands still, or walks at a speed lower than a threshold) during a second time period, and running again during a third time period, the first target interval may be a signal within the time period corresponding to the time period when the user is running, i.e., signals during the first time period and the third time period. In other words, the first target interval does not include signals within the time period when the user is taking a break, i.e., within the second time period.

In some embodiments, a plurality of first target intervals may be included in the action signal. For example, the signals within the first time period and the third time period described above may each be referred to as one first target interval.

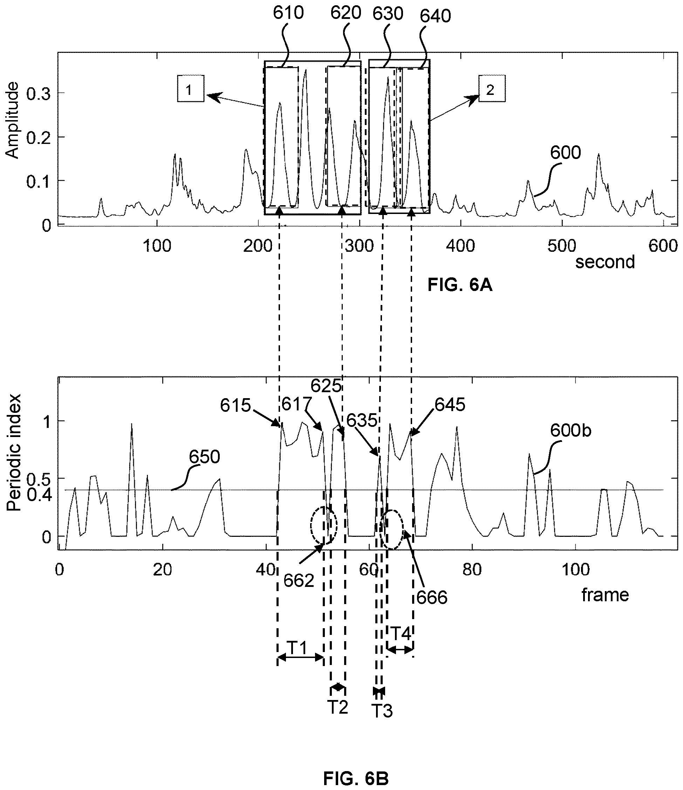

In the motions of physical exercise, most of the common motions, e.g., fitness, running, swimming, cycling, etc., are reciprocal motions. For example, when the user is running, the user needs to repeat the left and right legs staggered forward. As another example, when the user is doing push-ups, the user needs to repeat the bending and straightening of the arms. Periodic features may be present in the action signals of the reciprocal motions (e.g., as shown in FIG. 6A, signals in segment 1 and segment 2 of an action signal 600 are periodic). In some embodiments, the first target interval may also be referred to as a periodic segment in the action signal when the user is in a reciprocal motion (i.e., the target action may be the reciprocal motion). One target action may correspond to one motion cycle. For example, if the target action is a running action, the time it takes from the left leg stepping forward (after stepping forward through the right leg) to the left leg stepping forward again may be referred to as a motion cycle, and the corresponding action during that time may be referred to as the target action. As another example, if the target action is a push-up action, the time it takes for the arm to straighten (bend through the arm) until the arm straightens again may be referred to as a motion cycle, and the corresponding action during that time may be referred to as the target action. In some embodiments, the periodic segment (i.e., the first target interval) may include signals of a plurality of motion cycles of the target action. In other words, the periodic segment may reflect how many target actions the user has done in its corresponding time period.

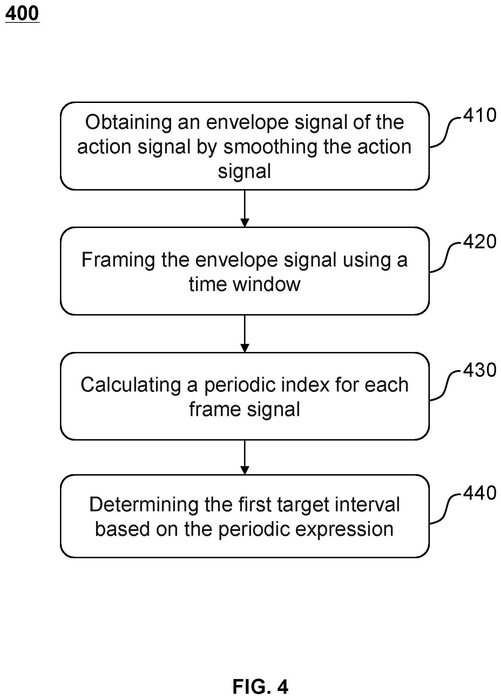

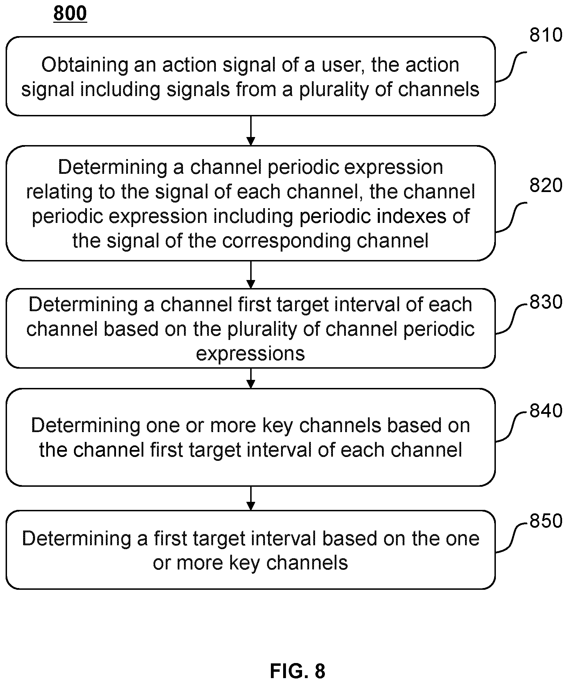

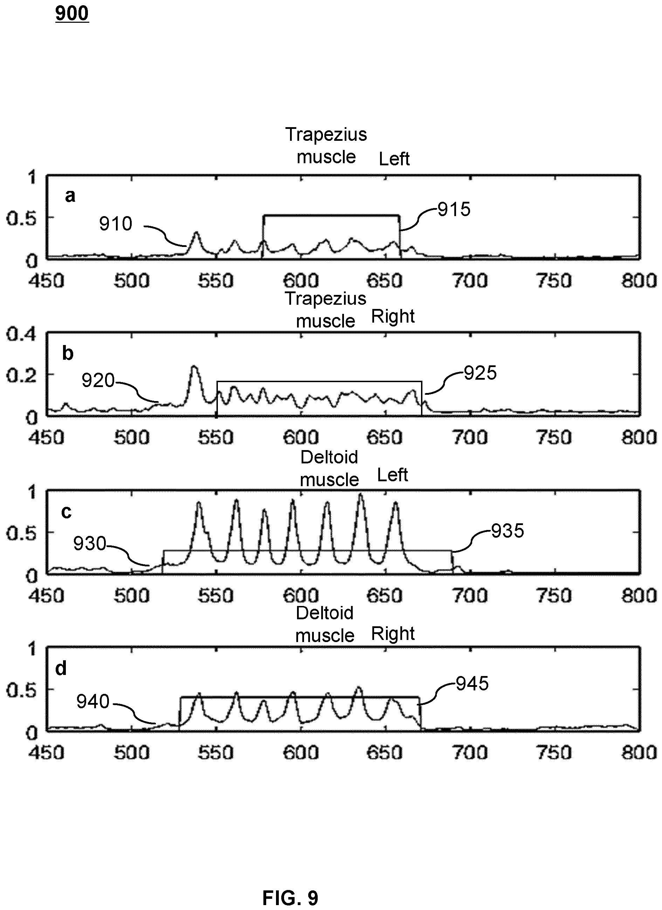

In some embodiments, the processing device 110 may determine one or more key channels based on the action signal. The processing device 110 may determine the first target interval based on the one or more key channels. Specifically, the processing device 110 may determine the first target interval based on a periodic expression relating to a signal of each key channel. In some embodiments, when the action signal includes a signal from only one channel, the processing device 110 may directly identify that channel as a key channel. That is, the processing device 110 may determine the first target interval based on the periodic expression relating to the action signal. More descriptions regarding identifying the first target interval in the case of one channel may be found in FIG. 4 and related descriptions thereof. At this point, the first target interval may be at least a portion of the signal in the channel. In some embodiments, when the action signal includes signals from a plurality of channels, the processing device 110 may select one or more of the plurality of channels to be key channels. At this time, the first target interval may include at least a portion of a signal of each of the one or more channels. For example, when the target action is a running action, the wearable device 130 may acquire the electromyography signals of the quadriceps femoris muscle, the electromyography signals of the biceps femoris muscle, the electromyography signals of the gastrocnemius muscle, and the electromyography signals of the gluteus maximus muscle of the left and right sides, i.e., the action signal of running may include signals from eight channels. At this point, the first target interval may include a union of channel first target intervals of the 8 channels. More descriptions regarding determining the first target interval that includes the signals of the plurality of channels may be found in FIG.8, FIG.9 and related descriptions thereof.

In some embodiments, the processing device 110 may determine the first target interval utilizing a trained machine learning model. For example, the processing device 110 may obtain the trained machine learning model and input the action signal into the trained machine learning model. The trained machine learning model may output the first target interval. As another example, the processing device 110 may first extract signal features (e.g., the periodic expression of the action signal and a period of each frame signal) of the action signal, and then input the signal features into the trained machine learning model to determine the first target interval. Correspondingly, when training the machine learning model, the extracted signal features may be directly inputted into the machine learning model as training samples, which reduces the complexity of the machine learning model and improves the robustness of the trained machine learning model when the training samples are few. In some embodiments, the trained machine learning model may include a convolutional neural network model (CNN), a deep convolutional neural network model (DCNN), a full convolutional neural network model (FCN), a recurrent neural network model (RNN), or the like, or variants and combinations thereof.

In 330, the processing device 110 may extract, from the first target interval, a second target interval that is capable of reflecting at least one motion cycle of the target action. In some embodiments, operation 330 may be performed by the second target interval extraction module 230.

The second target interval may include a signal of at least one motion cycle of the target action. In some embodiments, the second target interval may correspond to a force exerting starting (time) point and a force exerting ending (time) point of the at least one motion cycle of the target action (i.e., one target action). For example, if the target action is a push-up action, a time point when the user's arm bends may be used as the force exerting starting point of the biceps muscle, and a time point when the user's arm is extended may be used as the force exerting ending point of the biceps muscle. In some embodiments, due to the possibility that the target action of the user may not be completely identical, the first target interval may include different second target intervals. For example, one second target interval has a duration of 1.8 seconds, while another second target interval has a duration of 2 seconds. As another example, an amplitude of an amplitude-maximum point in one second target interval is smaller than an amplitude of an amplitude-maximum point in another second target interval.

In some embodiments, the processing device 110 may determine one or more key points in the first target interval and determine the second target interval based on the one or more key points. Since the first target interval may correspond to one or more key channels, the key points may be points on the signals of the one or more channels.

In some embodiments, when the action signal includes a signal of only one channel, i.e., the key point is only on a signal of one channel, the processing device 110 may derive a function corresponding to the signal of the first target interval and compute one or more maximum points of the function. In other words, the processing device 110 may determine one or more peak points of the signal in the first target interval. The processing device 110 may designate each maximum point that satisfies a certain condition (e.g., a corresponding magnitude exceeding a threshold) as a key point. Further, the processing device 110 may use each key point as a reference point (e.g., a center) and look around each key point (e.g., in a time period of 0.25 times the period before and after) for a force exerting starting point and a force exerting ending point corresponding to each key point. Specifically, the processing device 110 may determine a time point corresponding to a point closest to the key point that satisfies a first amplitude and/or period condition and is located before the key point as the force exerting starting point corresponding to the key point. The processing device 110 may determine a time point corresponding to a point closest to the key point that satisfies a second amplitude and/or period condition and is located after the key point as the force exerting ending point corresponding to the key point. In some embodiments, the first amplitude and/or period condition and the second amplitude and/or period condition may be the same or different. For example, for a particular key point with an amplitude of A, the processing device 110 may look around the key point for points with an amplitude that is a certain multiple of the amplitude A of the key point (e.g., a point with an amplitude of 0.5A). The processing device 110 may determine the first one point that satisfies the amplitude condition and is located before that key point as the force exerting starting point corresponding to that key point, and determine the first one point that satisfies the amplitude condition and is located after that key point as the force exerting ending point corresponding to that key point. As another example, the processing device 110 may determine a period (which may also be referred to as an interval period) corresponding to the first target interval. In the present disclosure, a period corresponding to an interval may refer to a period calculated by treating the interval as a frame signal. For a first target interval with an interval period of T, the processing device 110 may designate a key point as a reference point, and take a point that has a certain distance (e.g., 0.25 T) forward from the key point as a force exerting starting point corresponding to that key point. The processing device 110 may take a point that has another distance (e.g., 0.2 T) back from the key point as the force exerting ending point corresponding to the key point.

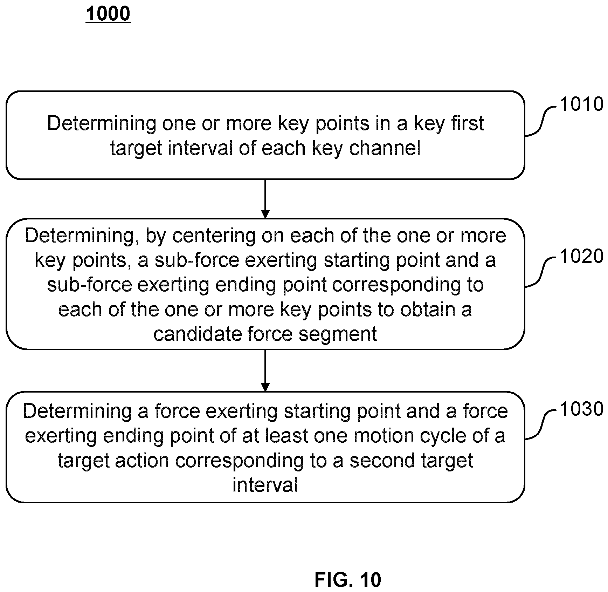

In some embodiments, when the action signal includes signals from the plurality of channels, the processing device 110 may determine a peak point of the signal of each key channel in the first target interval. The processing device 110 may select key points from the determined peak points based on a preset condition (e.g., the amplitude condition and the period conditions). More descriptions regarding extracting the second target interval may be found in FIG. 10 and related descriptions thereof.

In 340, the processing device 110 may evaluate the target action based on the second target interval. In some embodiments, operation 340 may be performed by the evaluation module 240.

In some embodiments, the evaluating the target action may include determining a count of actions of the user, identifying a motion type of the target action, analyzing whether the target action is standard, determining a percentage of muscle consumption, analyzing muscle activation timing and whether the muscle force exertion is standard, analyzing whether the user is fatigued, etc.

In some embodiments, when identifying the motion type of the target action, the processing device 110 may determine the motion type of the target action based on features such as a length of the second target interval (i.e., a duration corresponding to the second target interval) and/or a change in signal amplitude. Specifically, for various actions, the duration and/or signal amplitude of one of the actions corresponding thereto may be different. For example, compared to a running action, a leg of a weightlifting action corresponds to a larger signal amplitude, and the duration of the second target interval corresponding the weightlifting action may be longer than the duration of the second target interval corresponding to the running action. The database may have pre-stored duration ranges and/or signal amplitude ranges, etc., of second target intervals corresponding to various motion types. The processing device 110 may match the duration and/or signal amplitude of the second target intervals with various duration ranges and/or signal amplitude ranges in the database. The processing device 110 may determine a motion type corresponding to a duration range in which the duration of the second target interval is located and an amplitude range in which the amplitude is located as the motion type of the target action. In some embodiments, when the action signal includes signals from the plurality of channels, the processing device 110 may determine the motion type of the target action in conjunction with a muscle position, a force exertion amplitude of each muscle, and a force exertion duration and/or a force exertion sequence of each muscle corresponding to each channel (e.g., the key channel). Specifically, the database may have pre-stored muscle position, the force exertion amplitude of each muscle, the force exertion duration, and the force exertion sequence of each muscle, or the like, corresponding to each motion type. The processing device 110 may match the muscle position, the force exertion amplitude, the force exertion duration, and the force exertion sequence of each muscle corresponding to each channel (e.g., the key channel) with the muscle positions, the force exertion amplitudes, the force exertion durations, and the force exertion sequences of muscles stored in the database. The processing device 110 may determine a motion type that corresponds to the matched closest muscle position, the closest force exertion amplitude, the closest force exertion duration, and the closest force exertion sequence of a muscle as the motion type corresponding to the target action.

In some embodiments, whether an action is standard may include whether the force exertion sequence (or activation sequence) and/or a force exertion intensity of each muscle corresponding to the action is standard. In some embodiments, when analyzing whether the target action is standard the processing device 110 may determine, based on the motion type of the target action (e.g., determined based on the name of the motion entered by the user before exercising), whether the force exertion sequence of each muscle (or the corresponding muscle of the key channel) matches the standard/suggested force exertion sequence of the target action, thereby determining whether the target action is standard. In some embodiments, the processing device 110 may determine whether the target action is standard based on an amplitude of each key point in the first target interval. For example, when the amplitude of each key point exceeds a threshold, the processing device 110 may determine whether the target action is standard based on whether a difference (e.g., a variance, a standard variance, etc.) in amplitudes between key points is less than a difference threshold. When the difference in amplitudes between the key points is less than the difference threshold, the processing device 110 may determine that the target action is standard. In some embodiments, the processing device 110 may determine whether the target action is standard based on a percentage of muscle exertion corresponding to each key channel in the first target interval and/or the second target interval. The processing device 110 may determine whether the percentage of muscle exertion corresponding to each key channel matches a standard/recommended percentage of muscle exertion, and thus determine whether the target action is standard. In some embodiments, the processing device 110 may compare the amplitude of the key point in the second target interval corresponding to each key channel to determine the percentage of muscle exertion corresponding to each key channel. In some embodiments, the processing device 110 may determine an amplitude average of amplitudes of key points of the signal of each key channel (or key muscle) in the first target interval. The processing device 110 may determine the percentage of muscle exertion of each key channel based on the amplitude average of each key channel.

In some embodiments, when determining muscle consumption, the processing device 110 may determine the percentage of muscle exertion of each key channel determined based on the amplitude average of each key channel as the corresponding percentage of muscle consumption.

In some embodiments, when analyzing whether the user is fatigued, the processing device 110 may determine whether the user is fatigued based on a change in signal frequencies of a plurality of second target intervals in the first target interval. For example, when the signal frequencies of the plurality of second target intervals gradually decrease over time, the processing device 110 may determine that the user is fatigued. In some embodiments, the processing device 110 may also determine whether the user is fatigued based on the change in frequencies of signals in the plurality of first target intervals. For example, the processing device 110 may determine that the user is fatigued when a difference between a frequency of a signal in the last first target interval and a frequency of a signal in the penultimate first target interval is greater than a threshold. In some embodiments, the processing device 110 may also determine whether the user is fatigued based on the change in the amplitude of the signal in the first target interval and/or in conjunction with the information such as the heart rate, respiratory rate, etc. For example, for the electromyography signal, when the amplitude of the signal in the first target interval gradually increases, it may be determined that the muscle fatigue of the user gradually occurs.

In some embodiments, the processing device 110 may also evaluate the target action of the user using an action evaluation model based on the action signal or signal features corresponding to the action signal (e.g., the first target interval, the second target interval, the periodic expression of the action signal, and the period of the frame signal). In some embodiments, the action evaluation model may be constructed based on one or more of a linear classification model (LR), a support vector machine model (SVM), a plain Bayesian model (NB), a k-nearest neighbor model (KNN), a decision tree model (DT), an integration model (RF/GDBT, etc.), or the like. In some embodiments, the action evaluation model may include one or more machine learning models. In some embodiments, the action evaluation model may include, but is not limited to, a machine learning model for classifying the action signal of the user, a machine learning model for analyzing whether the action of the user is standard, a machine learning model for determining muscle consumption, a machine learning model for determining the count of actions of the user, and a machine learning model for analyzing a fatigue index of the user when performing the action. Different machine learning models may have different evaluation results. For example, the machine learning model for classifying the action signal of the user may take the action signal of the user as input data and output a corresponding motion type. As another example, the machine learning model for analyzing whether the action of the user is standard may take the action signal of the user as input data and thus output the quality of the action (e.g., standard action, wrong action). As yet another example, the machine learning model for analyzing the fatigue index of the user when performing the action may take the action signal (e.g., a frequency of the electromyography signal) of the user as input data and thus output the fatigue index of the user. In some embodiments, the action signal of the user and an evaluation result (an output) of the machine learning model may also be designated as sample data for training the action evaluation model, and the action evaluation model may be trained to optimize relevant parameters of the action evaluation model. It should be noted that the action evaluation model is not limited to the trained machine learning model described above, but may also be a preset model, e.g., a manually preset conditional judgment algorithm or a model determined by manually adding parameters (e.g., a confidence level) to the trained machine learning model, etc.

In some embodiments, the processing device 110 may send a feedback instruction to the wearable device 130 and/or the terminal device 140 based on the evaluation result of the target action. The wearable device 130 and/or the terminal device 140 may provide feedback to the user based on the feedback instruction. In some embodiments, the feedback may include emitting an alert message (e.g., a text message, a picture message, a video message, a voice message, an indicator message, etc.) and/or performing a corresponding action (by way of electrical current stimulation, vibration, pressure change, heat change, etc.) to stimulate the user's body. For example, when the user does sit-ups, the action signal of the user is monitored to be that the trapezius muscle exerts too much force (i.e., actions of the user's head and neck are not standard during the motion), in such cases, an output module (e.g., a vibration prompt) in the wearable device 130 and the terminal device 140 (e.g., a smartwatch, a smartphone, or the like) may perform corresponding feedback actions (e.g., applying a vibration to the user's body part, emitting a voice prompt, etc.) to prompt the user to adjust the force-exertion part promptly. In some embodiments, the processing device 110 may perform real-time monitoring of the action signal during the motion of the user. When the motion type, the quality of action, the count of actions, or the like, of the user during motion are determined, the terminal device 140 may output a corresponding record of action so that the user may understand his or her motion situation during motion.

It should be noted that the foregoing description of the process 300 is intended to be exemplary and illustrative only and does not limit the scope of application of the present disclosure. For a person skilled in the art, various corrections and changes may be made to the process 300 under the guidance of the present disclosure. However, these corrections and changes remain within the scope of the present disclosure. In some embodiments, the various models (e.g., the action evaluation model) addressed in the above descriptions may be trained by processing devices other than the processing device 110. In some embodiments, the processing device 110 may also not be predicated on identifying the first target interval, and may directly identify the action signal to determine the second target interval.

FIG. 4 is a flowchart illustrating an exemplary process for determining a first target interval according to some embodiments of the present disclosure. In some embodiments, process 400 may be performed by the first target interval identification module 220. As shown in FIG. 4, the process 400 may include the following operations.

In 410, the processing device 110 may obtain an envelope signal of an action signal by smoothing the action signal.

The smoothing process may attenuate the effects of interfering signals. In some embodiments, the smoothing process may include one or more of a filtering process, a convolution process, a burr signal removal process, a signal correction process, or the like. For example, the processing device 110 may determine one or more burr signals and/or discontinuous signals in the action signal (e.g., an electromyography signal or a postural signal). The processing device 110 may remove the burr signals or mutant signals, or adjust their amplitudes based on surrounding signals. As another example, when the action signal is the electromyography signal, the processing device 110 may perform a low-pass filtering process on the electromyography signal after calculating values such as root-mean-square (RMS), integrated electromyography (IEMG), or the like of the electromyography signal. In some embodiments, the low-pass filtering process may be implemented by a low-pass filter. Exemplary low-pass filters may include inductor-capacitor (LC) passive filters, resistor-capacitor (RC) passive filters, resistor-capacitor (RC) active filters, passive filters including special components, or the like. In some embodiments, the passive filter including the special components may include one or more of a piezoelectric ceramic filter, a crystal filter, or an acoustic surface filter.

In 420, the processing device 110 may frame the envelope signal using a time window.

In some embodiments, the time window may be a window with a specific length of time (which may also be referred to as a window length). Using the time window is possible to select a signal within a specific time period in the envelope signal (or the action signal), and the length of the specific time period is equal to the window length. A signal of a specific length of time determined by the time window may be referred to as a frame signal. Signals within different time periods may be selected from the envelope signal by moving the time window, i.e., dividing the envelope signal into a plurality of frames.

In some embodiments, the processing device 110 may move the window at a preset stepping value to frame the envelope signal. The smaller the stepping value, the more accurate the detected periodic expression. In some embodiments, two adjacent frame signals may have overlapping portions (as shown in FIG. 6A for a third frame signal 630 and a fourth frame signal 640).

In some embodiments, the window length may be determined based on default settings of the motion monitoring system 100. In some embodiments, the window length may be determined based on user input. For example, the user may enter the window length directly via the terminal device 140. As another example, the user may enter the name of the motion he or she is about to do at the beginning of the motion. The processing device 110 may determine the window length based on the name of the motion entered by the user. Specifically, window lengths corresponding to different motions may be stored in a database. The processing device 110 may look up the window length that matches the name of the motion entered by the user.

It is important to know that to improve the accuracy of results (e.g., a periodic index) of subsequent calculations, the window length may be greater than at least one motion cycle of the target action and less than a length of a periodic segment. Since there are differences in the period and a duration (i.e., the length of a periodic segment) of a motion of different motion types, for example, a push-up exercise may last 5s and a running exercise may last 30min, users or engineers may count features of various motions and set different window lengths for different motions. The user may store the window lengths corresponding to different motions in the database for call by the processing device 110. Merely by way of example, for a general periodic motion, e.g., running, swimming, cycling, etc., their corresponding periodic segments may contain a plurality of actions, so their corresponding window lengths may be set relatively long (e.g., 15s, 20s, etc.). Whereas in some motions, e.g., fitness exercises (e.g., the push-up exercise), their corresponding periodic segments may contain only a few actions (e.g., within a range of 3-15), so their corresponding window lengths may be set relatively short (e.g., 3s, 5s, etc.).

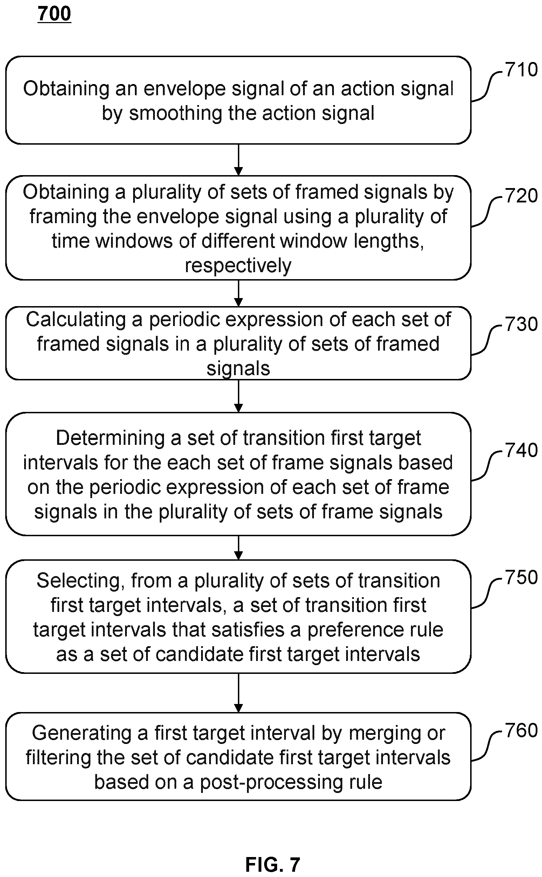

In some embodiments, when the motion type of the user is unknown, the processing device 110 may frame the envelope signal respectively using at least two time windows of different window lengths. More descriptions regarding processing the action signal using a plurality of time windows may be found in FIG. 7 and related descriptions thereof and may not be repeated here.

In 430, the processing device 110 may calculate a periodic index for each frame signal.

For the same time window, each frame signal may correspond to a periodic index. In other words, each periodic index may correspond to a segment of the action signal (which may also be referred to as a framed signal). The periodic index may reflect the periodicity and/or amplitude strength of the signal corresponding to the framed signal. The larger the value of the periodic index, the stronger the periodicity of the corresponding framed signal.

In some embodiments, the periodic index may be determined using a periodic function. In some embodiments, the periodic function may include a function (or algorithm) for finding/determining signal autocorrelation or signal amplitude difference. Exemplarily, the periodic function may include an autocorrelation function, a normalized autocorrelation function, a mean amplitude difference function, a normalized mean amplitude difference function, a periodic detection function based on frequency features, etc., and variants or any combination thereof. For example, the periodic function may be an autocorrelation function after clipping. As another example, the periodic function may be a cyclic mean amplitude difference function. As yet another example, the periodic function may be a new function formed by dividing an autocorrelation function by the mean amplitude difference function. It is to be appreciated that the periodic function may also be any other function capable of representing the periodicity, which is not beyond the scope of protection of the present disclosure. For illustrative purposes, the present disclosure will illustrate the amplitude difference function as an example of the periodic function. Exemplarily, the amplitude difference function (i.e., the periodic function) may be expressed as an equation (1) below:

$$D_n = \sum_{i=1}^{N-n}|x_i - x_{i+n}| \qquad (1),$$

where $i$ denotes the $i$-th signal point in a particular frame signal, $x_i$ denotes an intensity (or amplitude) of the $i$-th signal point, $N$ denotes a total count of signal points of the frame signal, and $n$ denotes a count of points at which the frame signal is misaligned.

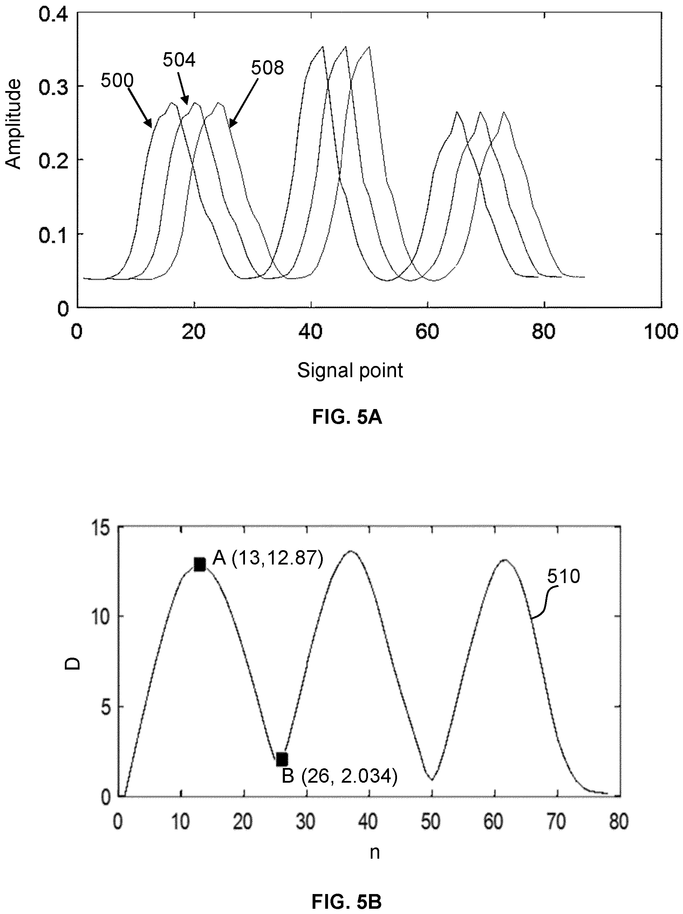

For each frame signal, the processing device 110 may obtain a curve (which may also be referred to as a periodic function curve) (as shown by curve 510 in FIG. 5B) corresponding to the periodic function of the frame signal based on the periodic function. The processing device 110 may determine a periodic index of the frame signal based on one or more curve features of the periodic function curve. In some embodiments, the curve features may include a peak point and a valley point of the periodic function curve. In some embodiments, the processing device 110 may determine the periodic index of the frame signal based on a difference between a longitudinal coordinate of the peak point and a longitudinal coordinate of the valley point of the periodic function curve. For example, as shown in FIG. 5B, the periodic function curve corresponds to a first peak point A having a longitudinal coordinate of 12.87 and a first valley point B having a longitudinal coordinate of 2.034, and the processing device 110 may determine the value of the periodic index of the frame signal corresponding thereto is 10.836. As another example, the processing device 110 may determine the difference in longitudinal coordinates between each two of adjacent peak point and valley point. The processing device 110 may determine any of an average, maximum, minimum, median, etc., of the difference value of the longitudinal coordinates as a periodic index of the frame signal.

Periodic indexes of all framed signals may form the periodic expression. The periodic expression may reflect a position of the first target interval (i.e., the periodic segment) in the action signal.

In some embodiments, the periodic expression may be represented as a curve, a table, a function, etc. For ease of description, the present disclosure may use a curve (as shown in curve 600b in FIG. 6B) as a representation of the periodic expression, which does not limit the scope of the present disclosure. In some embodiments, the periodic expression may be correlated with a window length, i.e., processing the same motion signal utilizing time windows with different window lengths may result in different periodic expressions. More descriptions regarding determining the periodic expression may be found in FIG. 8 and related descriptions thereof.

In some embodiments, the processing device 110 may also determine a period of the frame signal based on the curve features of the periodic function curve. Each periodic index and/or each frame signal may correspond to a period. In some embodiments, the processing device 110 may determine horizontal coordinates of the various valley points and/or peak points of the curve. The processing device 110 may determine, based on the horizontal coordinates of the valley points and/or peak points, a count of signal points corresponding to the period of the frame signal. The processing device 110 may convert the count of signal points to the period of the frame signal. For example, the processing device 110 may determine the period of the frame signal based directly on a horizontal coordinate of the first one valley point. As another example, the processing device 110 may determine the period of the frame signal based on a difference between the horizontal coordinates of two adjacent valley points (or peak points). As yet another example, the processing device 110 may determine the difference in the horizontal coordinates of every two adjacent valley points (or peak points). The processing device 110 may determine the period of the frame signal based on any of an average, a maximum, a minimum, a median, etc., of the differences in horizontal coordinates.

In 440, the processing device 110 may determine the first target interval based on the periodic expression.

In some embodiments, the processing device 110 may determine the first target interval based on a preset periodic rule. The processing device 110 may select one or more periodic indexes that satisfy the periodic rule from the periodic expression, and directly designate a signal segment corresponding to the selected periodic indexes as the first target interval. Specifically, the processing device 110 may designate a starting point of a certain frame signal corresponding to the first one periodic index that satisfies the periodic rule as a starting point of the first target interval, and designate an ending point of a certain frame signal corresponding to the last one periodic index that satisfies the periodic rule as an ending point of the first target interval to determine the first target interval. In some embodiments, the periodic rule may include that a width corresponding to continuous periodic indexes greater than a first threshold is greater than a first width threshold, a distance between adjacent two periodic indexes that are greater than the first threshold does not exceed a second width threshold (or a width corresponding to periodic indexes less than the first threshold between periodic indexes greater than the first threshold does not exceed the second width threshold), a difference in periods corresponding to two adjacent periodic indexes in a plurality of continuous periodic indexes is not greater than a first period threshold, a width corresponding to a distance between the first one periodic index and the last one periodic index that satisfies a first condition is greater than the first width threshold, a sum of signal segments corresponding to periodic indexes satisfying the first condition is greater than a first length threshold, etc.

In the present disclosure, a width corresponding to periodic indexes under a certain condition may correspond to a count of periodic indexes satisfying the certain condition. The width corresponding to the continuous periodic indexes greater than the first threshold refers to a count of continuous periodic indexes whose values are greater than the first threshold in the periodic expression. For example, as shown in FIG. 6B, if the first threshold is 0.4 (the longitudinal coordinate corresponding to curve 650), and points between point 615 and point 617 satisfy that their values are consecutively greater than 0.4, the count of periodic indexes between point 615 and point 617 is the width corresponding to the continuous periodic indexes greater than the first threshold. The first condition may include that the distance between adjacent two periodic indexes that are greater than the first threshold does not exceed the second width threshold and that the difference in the periods corresponding to the adjacent two periodic indexes is not greater than the first period threshold. The second width threshold may be less than the first width threshold.

In some embodiments, the periodic rule may be determined based on default settings of the motion monitoring system 100. In some embodiments, the periodic rule may be determined based on user input. For example, the user may determine the periodic rule by selecting the periodic rule via the terminal device 140 and entering relevant threshold data (e.g., the first threshold, the first width threshold, the second width threshold, the first length threshold, etc.).

In some embodiments, the processing device 110 may identify one or more candidate first target intervals from the action signal based on the periodic expression according to the periodic rule. Further, the processing device 110 may determine, for the signal in each candidate first target interval, a periodic index (which may also be referred to as an interval periodic index) and/or interval period corresponding to the candidate first target interval. In the present disclosure, a periodic index corresponding to an interval refers to a periodic index obtained by calculating using the interval as a frame signal.

The processing device 110 may generate the first target interval by merging or filtering the one or more candidate first target intervals based on a post-processing rule. In some embodiments, the post-processing rule may include a merging rule and/or a filtering rule. The merging rule may include two adjacent candidate first target intervals satisfying that a length between an ending point of the first one candidate first target interval and a starting point of the second one candidate first target interval is less than a second length threshold, or a difference between periods of two adjacent candidate first target intervals is less than a period threshold. The filtering rule may include in response to determining that a periodic index corresponding to a signal after merging the two adjacent candidate first target intervals is greater than a second threshold, determining a merged interval as the first target interval, or in response to determining that the periodic index corresponding to the signal after merging the two adjacent candidate first target intervals is less than the second threshold, performing no merging, i.e., each of the two adjacent candidate first target intervals is determined as one first target interval.

It should be noted that the foregoing description of the process 400 is intended to be exemplary and illustrative only and does not limit the scope of application of the present disclosure. For a person skilled in the art, various corrections and changes may be made to the process 400 under the guidance of the present disclosure. However, these corrections and changes remain within the scope of the present disclosure. In some embodiments, the processing device 110 may process the same set of framed signals at the same time using two different periodic functions to obtain two sets of periodic indexes (or periodic expressions) and periods. The processing device 110 may determine the first target interval based on the two sets of periodic indexes and/or periods according to a suitable periodic rule. For example, for the two sets of periodic expressions, the processing device 110 may determine a signal segment corresponding to periodic indexes that satisfy the width corresponding to continuous periodic indexes greater than the first threshold being greater than the first width threshold as the first target interval. As another example, the processing device 110 may determine a signal segment corresponding to periodic indexes that satisfy the difference between periods of the plurality of continuous periodic indexes is not greater than the first period threshold as the first target interval.

FIG. 5A is a schematic diagram illustrating an exemplary process for determining a periodic function curve of a frame signal according to some embodiments of the present disclosure. As shown in FIG. 5A, curve 500 represents a frame signal with periodicity. Curve 504 represents a signal after translating curve 500 horizontally by 4 points. Curve 508 represents a signal after translating curve 500 horizontally by 8 points.

From FIG.5 A, it can be seen that the signal 500 is a digital signal composed of 80 signal points. In some embodiments, the processing device 110 may calculate an amplitude difference function (i.e., a periodic function) of the frame signal 500 according to equation (1). In equation (1), $n$ denotes a count of points at which the signal is misaligned. Equivalently, the curve 500 is translated by different counts of points, such as curves 504 and 508. In the process of calculating the periodic function curve of signal 500, $n$ may take different positive integers ($n$ is less than or equal to $N$) (e.g., 1, 2, ......, 80). For example, the processing device 110 may calculate the value of $D_1$ by translating the curve 600 by 1 point (at this point, the value of n in equation (1) is 1). Similarly, the processing device 110 may calculate the values of $D_2$, $D_3$, ......, and D80, respectively. Taking a count of misaligned points corresponding to $D_1$, $D_2$, $D_3$, ......, and $D_{80}$ as horizontal coordinates, and the values thereof as longitudinal coordinates, thus obtaining a periodic function curve of the frame signal 500, as shown in FIG.5 B.

FIG. 5B is a schematic diagram illustrating a periodic function curve of the signal 500 in FIG. 5A according to some embodiments of the present disclosure. As shown in FIG.5B, the periodic function curve 510 may have a plurality of peak points and valley points. In some embodiments, the processing device 110 may determine a periodic index corresponding to the signal 500 based on longitudinal coordinates of the peak points and the valley points and determine a period of the signal 500 based on horizontal coordinates of the peak points and/or the valley points. For example, the processing device 110 may determine a difference (i.e., 10.836) between a longitudinal coordinate of a peak point A (i.e., 12.87) and a longitudinal coordinate of a valley point B (i.e., 2.034) as the periodic index of the signal 500. The processing device 110 may determine a length in the signal 500 of the count of points (i.e., 26) corresponding to the horizontal coordinate of the valley point B as the period of the signal 500.

FIG. 6A is a schematic diagram illustrating an action signal according to some embodiments of the present disclosure. FIG. 6B is a periodic expression of the action signal in FIG. 6A. As shown in FIG. 6A and FIG. 6B, an action signal 600 is a segment of a signal that is locally periodic. A window length of a time window is 6 seconds.

The processing device 110 may obtain various frame signals by framing the action signal 600 using the time window, for example, a first frame signal 610, a second frame signal 620, a third frame signal 630, and a fourth frame signal 640. Further, the processing device 110 may calculate a periodic index and/or period of each frame signal using a periodic function. For example, the periodic index of the first frame signal 610 is a first periodic index 615, the periodic index of the second frame signal 620 is a second periodic index 625, the periodic index of the third frame signal 630 is a third periodic index 635, and the periodic index of the fourth frame signal 640 is a fourth periodic index 645. All the periodic indexes may form a periodic expression 600b.

The processing device 110 may determine the first target interval based on a preset periodic rule. For example, the processing device 110 may determine the signal that corresponds to periodic indexes satisfy a width corresponding to continuous periodic indexes greater than a first threshold (e.g., a longitudinal coordinate of 0.4 corresponding to the curve 650) being greater than the first width threshold as a periodic segment. For example, if widths corresponding to intervals T1, T2, and T4 are greater than the first width threshold, the processing device 110 may determine the signals corresponding to the periodic indexes in the intervals T1, T2, and T4 as the first target intervals. As another example, since the width 662 corresponding to the periodic indexes below 0.4 (i.e., the first threshold) between the intervals T1 and T2 is less than the second width threshold, the processing device 110 may merge a signal interval corresponding to T1 with a signal interval corresponding to T2 to obtain a relatively long first target interval (i.e., a periodic segment 1). Since the width 666 of the periodic indexes below 0.4 (i.e., the first threshold) between the intervals T3 and T4 is less than the second width threshold, the processing device 110 may merge a signal interval corresponding to T3 with a signal interval corresponding to T4 to obtain another relatively long first target interval (i.e., a periodic segment 2). In some embodiments, the processing device 110 may further determine whether to merge the periodic segment 1 and the periodic segment 2. For example, the processing device 110 may calculate an overall periodic index for an entire segment of signals from a starting point of the periodic segment 1 to an ending point of the periodic segment 2. If the overall periodic index is greater than the second threshold, the merged interval is determined as the first target interval. If the overall periodic index is less than the second threshold, then the periodic segment 1 and the periodic segment 2 are respectively determined as the first target interval.

FIG. 7 is a flowchart illustrating an exemplary process for determining a first target interval according to some embodiments of the present disclosure. Process 700 may be used to determine a first target interval when processing an action signal utilizing a plurality of time windows. In some embodiments, the process 700 may be performed by the first target interval identification module 220. As shown in FIG. 7, the process 700 may include the following operations.

In 710, the processing device 110 may obtain an envelope signal of the action signal by smoothing the action signal. Operation 710 may be performed in a similar manner to operation 410, which is not described herein.

In 720, the processing device 110 may obtain a plurality of sets of framed signals by framing the envelope signal using a plurality of time windows of different window lengths, respectively.

Since the result of calculating a periodic expression relating to the action signal may be related to the window length of the time window, when a target action of a user is unknown, or in the case where the user is doing multiple different motions in a single exercise, it is not possible to obtain an accurate periodic expression at once to obtain an accurate periodic segment for the processing device 110 by setting a suitable window length. In such cases, the accuracy of the motion monitoring system 100 can be improved by processing a plurality of time windows with different window lengths separately, and then finally selecting the accurate periodic segment according to a preference rule.

For illustrative purposes only, the plurality of time windows with different window lengths may include a first time window and a second time window. In some embodiments, window lengths of the first time window and the second time window may differ significantly in order to detect more motions with fewer time windows. For example, when the time length of the first time window is 3s, the time length of the second time window may be 20s. In some embodiments, different time windows may correspond to different stepping values. The stepping value for a time window with a shorter window length may be larger than the stepping value for a time window with a longer window length. In some embodiments, the processing of the action signal utilizing the plurality of time windows with different window lengths may be performed in parallel to reduce processing time.

In 730, the processing device 110 may calculate a periodic expression of each set of framed signals in the plurality of sets of framed signals.

The periodic expression of each set of framed signals may be determined in a similar manner to operation 430 and is not described herein.

In 740, the processing device 110 may determine a set of transition first target intervals for each set of frame signals based on the periodic expression of each set of frame signals in the plurality of sets of frame signals.

In some embodiments, the processing device 110 may select the transition first target intervals for each set of frame signals based on the periodic rule. More detailed descriptions regarding the periodic rule may be found in operation 440 and its description, which may not be repeated here.

In 750, the processing device 110 may select, from a plurality of sets of transition first target intervals, a set of transition first target intervals that satisfies the preference rule as a set of candidate first target intervals.

In some embodiments, the preference rule may include a sum of interval periodic indexes being maximum, the set of transition first target intervals having the most forward starting point, etc. For example, for a signal in each set of transition first target intervals of the plurality of sets of transition first target intervals, the processing device 110 may determine its corresponding interval periodic index and/or interval period. The processing device 110 may determine a sum of interval periodic indexes of the transition first target intervals in each set of transition first target intervals. The processing device 110 may select a set of transition first target intervals with the sum of interval periodic indexes being maximum as a set of candidate first target intervals. As another example, the processing device 110 may select a set of transition first target intervals with a more forward starting point as a set of candidate first target intervals.

In some embodiments, the processing device 110 may also select one or more candidate first target intervals from a plurality of sets of transition first target intervals. That is, the candidate first target intervals may include transition first target intervals in different sets. Exemplarily, the plurality of sets of transition first target intervals may include a first set of transition first target intervals and a second set of transition first target intervals. When there exists a transition first target interval in the first set of transition first target intervals that do not overlap with a transition first target interval in the second set of transition first target intervals, the processing device 110 may determine the transition first target interval in the first set as the candidate first target interval. Similarly, when there exists a transition first target interval in the second set of transition first target intervals that do not overlap with the first set of transition first target intervals, the processing device 110 may determine the transition first target interval in the second set as the candidate first target interval. For two transition first target intervals that are located in the first set of transition first target intervals and the second set of transition first target intervals, respectively, and that have overlapping portions, the processing device 110 may select the transition first target interval that has a larger interval periodic index as the candidate first target interval.

In 760, the processing device 110 may generate the first target interval by merging or filtering the set of candidate first target intervals based on a post-processing rule.

More descriptions regarding merging and filtering the candidate first target intervals may be found in operation 440 and its description, which is not repeated here.

It should be noted that the foregoing description of the process 700 is intended to be exemplary and illustrative only and does not limit the scope of application of the present disclosure. For a person skilled in the art, various corrections and changes can be made to the process 700 under the guidance of the present disclosure. However, these corrections and changes remain within the scope of the present disclosure. In some embodiments, the processing device 110 may select, from the periodic expressions of the plurality of sets of framed signals, periodic expressions that satisfy a preset condition as a target periodic expression relating to the action signal. The processing device 110 may select the first target interval from the action signal based on the target periodic expression. The preset condition may include the first occurrence of that a width corresponding to continuous periodic indexes greater than the first threshold being greater than the first width threshold, or a sum (or average) of values of periodic indexes that satisfy the width corresponding to continuous periodic indexes greater than the first threshold being greater than the first width threshold.

FIG. 8 is a flowchart illustrating an exemplary process for determining a first target interval according to some embodiments of the present disclosure. Process 800 may be used to determine the first target interval when an action signal includes signals from a plurality of channels. In some embodiments, the process 800 may be performed by the first target interval identification module 220. As shown in FIG. 8, the process 800 may include the following operations.

In 810, the processing device 110 may obtain the action signal of a user. The action signal may include signals from the plurality of channels.

When the user is in motion, a plurality of muscles are often required to work together to accomplish the corresponding action. Thus, in some embodiments, in order to enable the obtained action signal can reflect a target action of the user more accurately, and thus enhancing the accuracy of the final detection result, the processing device 110 may control a plurality of sensors in the wearable device 130 simultaneously collect signals from a plurality of different muscles. The signal obtained by each sensor is a signal of one channel. The signal of each channel may correspond to one type of force exertion muscle. In other words, one type of muscle may correspond to one channel. For example, as shown in FIG. 9, an action signal 900 may include signals of four channels, namely a signal 910 of a channel *a* corresponding to the left trapezius, a signal 920 of a channel *b* corresponding to the right trapezius, a signal 930 of a channel *c* corresponding to the left deltoid muscle, and a signal 930 of a channel d corresponding to the right deltoid. More description regarding the action signals may be found in operation 310 and its description.

In 820, the processing device 110 may determine a channel periodic expression relating to the signal of each channel. The channel periodic expression may include periodic indexes of the signal corresponding to the channel.

The channel periodic expression refers to a periodic expression relating to a signal of a certain channel. The signal of each channel may correspond to a channel periodic expression. The processing device 110 may determine the channel periodic expression of each channel separately. For example, the processing device 110 may frame the signal of each channel using a time window and calculate the periodic indexes of each frame signal, separately. All the periodic indexes corresponding to framed signals of a channel form a channel periodic expression of the channel. More description regarding determining the periodic expression of the signal of each channel may be found elsewhere in the present disclosure (e.g., FIG. 4, FIG. 7, and their descriptions).

In 830, the processing device 110 may determine a channel first target interval of each channel based on the plurality of channel periodic expressions.

For the signal of each channel, the processing device 110 may select the channel first target interval for that channel utilizing channel periodic indexes of the channel based on a periodic rule. In some embodiments, the processing device 110 may designate signals selected based on the periodic rule as channel candidate first target intervals. The processing device 110 may merge some or all of the channel candidate first target intervals to generate the channel first target interval. It is to be appreciated that the selection of the channel first target interval may be performed in a similar manner as the selection of the first target interval described in operation 440, and will not be repeated herein.

In 840, the processing device 110 may determine one or more key channels based on the channel first target interval of each channel.

The key channel refers to a channel corresponding to a muscle that plays a major role in the process of the target action of the user. For example, for a fitness exercise, due to there being a plurality of types of the fitness motions, the body parts that are worked out are also complex and varied. However, the fitness exercise has characteristics that there are usually one or more key muscles that are targeted for exercise. The key muscles exert relatively greater force during exercising, and therefore channels corresponding to the key muscles may be determined as key channels. For example, the sit-up exercise mainly trains the rectus abdominis, external abdominal obliques, and internal abdominal obliques, and key channels may be channels corresponding to the rectus abdominis, external abdominal obliques, and internal abdominal obliques. As another example, in the running exercise, the main force is exerted on the quadriceps, biceps femoris, gastrocnemius, and gluteus maximus muscles, so channels corresponding to the quadriceps, biceps femoris, gastrocnemius, and gluteus maximus muscles on the left and right sides of the user, or part of these corresponding channels may be key channels.

In some embodiments, the processing device 110 may determine one or more key channels based on interval periodic indexes of the channel first target interval of each channel. For example, the processing device 110 may select a channel with the largest sum of interval periodic indexes as the key channel. As another example, the processing device 110 may select a channel with the sum of the interval periodic indexes greater than an index threshold as the key channel. In some embodiments, the processing device 110 may determine one or more key channels based on a signal amplitude of the channel first target interval of each of the plurality of channels. For example, the processing device 110 may select a channel with a channel first target interval having a signal amplitude exceeding a threshold as the key channel. As another example, the processing device 110 may select a channel with a channel first target interval having a signal amplitude exceeding a threshold and with a sum of the interval periodic indexes greater than the index threshold as the key channel.

In some embodiments, the processing device 110 may also determine one or more key channels directly based on a motion type of the target action. For example, each motion type may correspond to one or more key channels. The user may input the motion type of the target action at any point during the motion, and the processing device 110 may determine one or more key channels corresponding to the motion type of the target action based on the motion type of the target action. As another example, the wearable device 130 may be a wearable device that is targeted for a particular motion. The processing device 110 may directly determine the key channels based on the motion type targeted by the wearable device 130.

In some embodiments, after determining that a channel corresponding to a particular muscle is a key channel, the processing device 110 may determine that a channel symmetrical to that key channel is another key channel.

In some embodiments, the processing device 110 may also filter an initially determined key channels to further determine the key channel. For example, for three initially determined candidate key channels, each candidate key channel may have a channel first target interval. The processing device 110 may determine an interval period of the channel first target interval for each candidate key channel. The processing device 110 may determine two candidate key channels with similar interval periods as key channels.

In 850, the processing device 110 may determine the first target interval based on the one or more key channels.

In some embodiments, the processing device 110 may determine the channel first target interval (which may also be referred to as a key first target interval) corresponding to the key channels as the first target interval directly. In this case, the first target interval may include the key first target interval of each key channel.

In some embodiments, the processing device 110 may determine an intersection or a union between times corresponding to a plurality of key first target intervals as the first target interval. For example, for motions in which the left and right sides (e.g., left and right hands and/or left and right legs) are performing actions simultaneously, the processing device 110 may determine the intersection between times corresponding to the plurality of key first target intervals as the first target interval. As another example, for motions in which the left and right sides (e.g., left and right hands and/or left and right legs) are staggered to perform actions, the processing device 110 may determine the union between times corresponding to the plurality of key first target intervals as the first target interval. For example, when there are two key channels, the first key channel has a key first target interval that corresponds to a time of 5s-25s. The second key channel also has a key first target interval that corresponds to a time of 5.5 s-25.5s. The processing device 110 may determine, based on the union of the two time intervals, that the first target interval corresponds to a time of 5s-25.5s, i.e., that the first target interval includes signals at 5s-25.5s of the first channel and signals at 5s-25.5s of the second channel.

It should be noted that the foregoing description of the process 800 is intended to be exemplary and illustrative only and does not limit the scope of application of the present disclosure. For a person skilled in the art, various corrections and changes may be made to the process 800 under the guidance of the present disclosure. However, these corrections and changes remain within the scope of the present disclosure. In some embodiments, the processing device 110 may utilize time windows of different window lengths to frame different channels when framing signals from different channels.

FIG. 9 is a schematic diagram illustrating an action signal including signals of a plurality of channels according to some embodiments of the present disclosure. As shown in FIG. 9, an action signal 900 may include signals of 4 channels, i.e., channels a, b, c, and d. A signal 910 of the channel *a* corresponding to the left trapezius muscle, a signal 920 of the channel *b* corresponding to the right trapezius muscle, a signal 930 of the channel *c* corresponding to the left deltoid muscle, and a signal 940 of the channel d corresponding to the right deltoid muscle.

According to FIG. 9, it may be seen that the signals corresponding to channels *c* and *d* (i.e., left and right deltoid muscles) show obvious periodic features (segment 935 and segment 945 correspond to relatively large periodic indexes) and their corresponding amplitudes are large, whereas the signals corresponding to channels a and *b* (i.e., left and right trapezius muscles) have poorer periodic features (segment 915 and segment 925 correspond to relatively small periodic indexes) and their corresponding amplitudes are smaller. The processing device 110 may determine channels *c* and *d* as key channels.

FIG. 10 is a flowchart illustrating an exemplary process for determining a second target interval according to some embodiments of the present disclosure.

Process 1000 may be used to determine the second target interval when the action signal includes signals from a plurality of channels. In some embodiments, the process 1000 may be performed by the second target interval extraction module 230. As shown in FIG. 10, the process 1000 may include the following operations.

In 1010, the processing device 110 may determine one or more key points in a key first target interval of each key channel. The determination of the key points may be performed with reference to operation 330.

In 1020, the processing device 110 may determine, by centering on each of the one or more key points, a sub-force exerting starting point and a sub-force exerting ending point corresponding to each of the one or more key points to obtain a candidate force segment.

The sub-force exerting starting point and the sub-force exerting ending point refer to a time starting point and a time ending point, respectively, at which a muscle exerts a force during a motion cycle. The processing device 110 may determine the sub-force exerting starting point and the sub-force exerting ending point corresponding to the key point based on an interval period of the key first target interval and/or the amplitude of the corresponding signal. Each key point in the key first target interval may correspond to an interval period of that key first target interval.

In some embodiments, the processing device 110 may find forward, in the signal of each key channel, a time point corresponding to the first one point whose signal amplitude is a first threshold multiple of an amplitude of the corresponding key point (the first threshold multiple is less than or equal to 1) as the sub-force exerting starting point. The processing device 110 may find backward a time point corresponding to the first one point whose signal amplitude is a second threshold multiple of an amplitude of the corresponding key point (the second threshold multiple is less than or equal to 1) as the sub-force exerting ending point. The first threshold multiple and the second threshold multiple may be the same or different.

In some embodiments, the processing device 110 may designate the key point as a reference point and take forward a time point corresponding to a third threshold multiple (the third threshold multiple is less than or equal to 1 and, preferably, equal to 0.5) of the interval period of the key first target interval as the sub-force exerting starting point of the key point, for example, take forward a time point corresponding to 0.25T (T being the interval period) as the sub-force exerting starting point. The processing device 110 may take backward a time point corresponding to a fourth threshold multiple (the fourth threshold multiple is less than or equal to 1 and, preferably, equal to 0.5) of the interval period of the key first target interval as the sub-force exerting ending point of the key point, e.g., take backward a time point corresponding to 0.25T (T being the interval period) as the sub-force exerting ending point. The third threshold multiple and the fourth threshold multiple may be the same or different.

In 1030, the processing device 110 may determine, based on each candidate force exerting segment, a force exerting starting point and a force exerting ending point of at least one motion cycle of the target action corresponding to the second target interval.

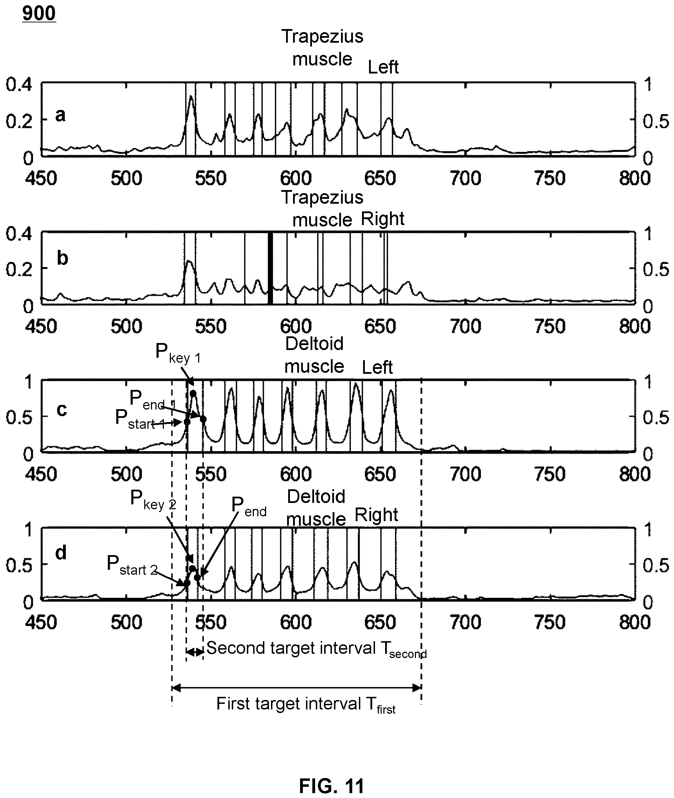

The force exerting starting point and the force exerting ending point refer to a motion starting point and a motion ending point of a target action, respectively. A signal in a time period between the force exerting starting point and the force exerting ending point is the second target interval. When the user is executing a target action, which requires the cooperation of various muscles, at this time, the force exerting starting point may be the sub-force exerting starting point of one muscle, and the corresponding force exerting ending point may be the sub-force exerting ending point of another muscle. Alternatively, the force exerting starting point and the force exerting ending point may be the sub-force exerting starting point and the sub-force exerting ending point of the same muscle. For example, as shown in FIG. 11, the force exerting starting point and force exerting ending point of the second target interval $T_{second}$ may be the force exerting starting point $P_{start\ 1}$ and sub-exerting ending point $P_{end\ 1}$ of the left deltoid (channel $c$).

In some embodiments, the processing device 110 may determine the force exerting starting point and the force exerting ending point based on a force exertion sequence (or activation order) of the force exertion muscles corresponding to the key channels. Specifically, the processing device 110 may combine candidate force segments that satisfy the force exertion sequence to determine the force exerting starting point and the force exerting ending point of at least one motion cycle of the target action corresponding to the second target interval. The processing device 110 may determine a sub-force exerting starting point of a candidate force segment that is ranked at the top of the order as the force exerting starting point and a sub-force exerting ending point of a candidate force segment that is ranked at the bottom of the order as the force exerting ending point. In some embodiments, the force exertion sequence of the force exertion muscles may be determined based on the order of the starting and ending points of the key first target intervals of the key channels. In some embodiments, the force exertion sequence of the force exertion muscles may be determined based on a motion type. Different motion types may have corresponding standard force exertion sequences.

In some embodiments, the processing device 110 may also determine the force exerting starting point and the force exerting ending point based on a signal of a non-key channel. For example, the processing device 110 may determine whether a peak point in the non-key channel (which may also be referred to as an auxiliary channel, and whose corresponding force exertion muscle may be a synergistic muscle) occurs near the key point of the key channel (e.g., 0.3T from the key point, where T is the interval period corresponding thereto). The processing device 110 may determine the force exerting starting point and/or the force exerting ending point of the target action based on the peak point of the auxiliary channel. For example, if the peak point of the auxiliary channel occurs before the key point, the processing device 110 may determine the peak point of the auxiliary channel as the force exerting starting point of the target action. If the peak point of the auxiliary channel occurs after the key point, the processing device 110 may determine the peak point of the auxiliary channel as the force exerting ending point of the target action. As another example, the processing device 110 may designate the peak point of the auxiliary channel as an auxiliary key point to determine an auxiliary force exerting segment corresponding to the peak point. If the peak point (i.e., the auxiliary key point) occurring in the auxiliary channel is before the key point, the processing device 110 may determine an auxiliary force exerting starting point corresponding to the peak point of the auxiliary channel as the force exerting starting point of the target action. If the peak point occurring in the auxiliary channel is after the key point, the processing device 110 may determine an auxiliary force exerting ending point corresponding to the peak point of that auxiliary channel as the force exerting ending point of the target action.

It should be noted that the foregoing description of the process 1000 is intended to be exemplary and illustrative only and does not limit the scope of application of the present disclosure. For a person skilled in the art, various corrections and changes may be made to the process 1000 under the guidance of the present disclosure. However, these corrections and changes remain within the scope of the present disclosure. In some embodiments, the processing device 110 may also utilize a trained machine learning model to determine the second target interval. The processing device 110 may input the action signal into the trained machine learning model. The trained machine learning model may output the force exerting starting point and the force exerting ending point of at least one motion cycle of the target action corresponding to the second target interval. As another example, the processing device 110 may first extract signal features of the action signal (e.g., a periodic expression of the action signal and a period of a frame signal, or a first target interval), and then input the signal features into the trained machine learning model to determine the second target interval.

FIG.11 is a schematic diagram illustrating a process of determining the second target interval of the signal 900 in FIG.9. As shown in FIG. 11, the processing device 110 may determine, in a first target interval $T_{first}$ of the signal 900, one or more key points in the key channels $c$ and $d$, respectively, such as key points $P_{key\ 1}$ and $P_{key\ 2}$. The processing device 110 may find forward and backward a sub-force exerting starting point and a sub-force exerting ending point corresponding to the key points, respectively. For example, the key point $P_{key\ 1}$

corresponds to a sub-force exerting starting point $P_{start\ 1}$ and a sub-force exerting ending point $P_{end\ 1}$, and the key point $P_{key\ 2}$ corresponds to a sub-force exerting starting points $P_{start\ 2}$ and a sub-force exerting ending point $P_{end\ 2}$. $P_{start\ 1}$ and $P_{start\ 2}$ may correspond to the same time point. In other words, the muscles corresponding to channels $c$ and $d$ may exert force at the same time when in motion. The processing device 110 may determine a time period corresponding to the time period between $P_{start\ 1}$ and $P_{end\ 1}$ as one candidate force exertion segment and a time period corresponding to the time period between $P_{start\ 2}$ and $P_{end\ 2}$ as another candidate force exertion segment. The processing device 110 may determine a union of the two candidate force exertion segments as the second target interval. In other words, the second target interval may include the signal from the time period between $P_{start\ 1}$ and $P_{end\ 1}$ in the channel $c$ and the signal from the time period between $P_{start\ 2}$ and $P_{end\ 2}$ in the channel $d$. The time point corresponding to $P_{start\ 1}$ or $P_{start\ 2}$ is the force exerting starting point of one motion cycle of the target action, and the time point corresponding to $P_{end\ 1}$ is the force exerting ending point of one motion cycle of the target action.

Having thus described the basic concepts, it may be rather apparent to those skilled in the art after reading this detailed disclosure that the foregoing detailed disclosure is intended to be presented by way of example only and is not limiting. Although not explicitly stated here, those skilled in the art may make various modifications, improvements, and amendments to the present disclosure. These alterations, improvements, and amendments are intended to be suggested by this disclosure and are within the spirit and scope of the exemplary embodiments of the present disclosure.

**Claims**

1. A method for motion monitoring, comprising:

    obtaining an action signal of a user;
    identifying a first target interval in the action signal, the first target interval corresponding to a target action of the user;
    extracting, from the first target interval, a second target interval that is capable of reflecting at least one motion cycle of the target action; and
    evaluating the target action according to the second target interval.

2. The method of claim 1, wherein the action signal includes an electromyographic signal or a postural signal.

3. The method of claim 2, wherein the identifying a first target interval in the action signal includes:

    determining a periodic expression relating to the action signal; and
determining the first target interval at least based on the periodic expression.

4. The method of claim 3, wherein the determining a periodic expression relating to the action signal includes:

    obtaining an envelope signal of the action signal by smoothing the action signal;
    framing the envelope signal using a time window; and
    calculating a periodic index for each frame signal, all periodic indexes forming the periodic expression.

5. The method of claim 4, wherein the calculating a periodic index for each frame signal includes:
for each frame signal,

    obtaining a periodic function;
    determining a periodic function curve corresponding to the frame signal based on the periodic function; and
    determining the periodic index of the frame signal based on one or more curve features of the periodic function curve.

6. The method of claim 5, wherein the one or more curve features include a peak point and a valley point of the periodic function curve.

7. The method of claim 5, further comprising:
determining a period of the frame signal based on the one or more curve features of the periodic function curve.

8. The method of claim 3, wherein the determining the first target interval at least based on the periodic expression includes:

    identifying, based on the periodic expression, at least one candidate first target interval from the action signal according to a periodic rule; and
    generating the first target interval by merging or filtering the at least one candidate first target interval based on a post-processing rule.

9. The method of claim 8, wherein the periodic rule includes at least one of:

    a width corresponding to continuous periodic indexes greater than a first threshold being greater than a first width threshold,
    a width corresponding to a distance between a first periodic index and a last periodic index that satisfy a first condition being greater than the first width threshold, wherein the first condition includes that a distance between adjacent two periodic indexes that are greater than the first threshold does not exceed a second width threshold and a difference in periods corresponding to the adjacent two periodic indexes is not greater than a first period threshold, or

a sum of signal segments corresponding to periodic indexes satisfying the first condition being greater than a first length threshold.

10. The method of claim 8, wherein the post-processing rule includes a merging rule, and the merging rule includes:

two adjacent candidate first target intervals satisfying that a length between an ending point of a first candidate first target interval and a starting point of a second candidate first target interval is less than a second length threshold, or

a difference between periods of two adjacent candidate first target intervals is less than a period threshold.

11. The method of claim 10, wherein the post-processing rule further includes a filtering rule, and the filtering rule includes:

in response to determining that a periodic index corresponding to a signal after merging the two adjacent candidate first target intervals is greater than a second threshold, determining a merged interval as the first target interval; or

in response to determining that the periodic index corresponding to the signal after merging the two adjacent candidate first target intervals is less than the second threshold, performing no merging.

12. The method of claim 2, wherein the identifying a first target interval in the action signal includes:

obtaining an envelope signal of the action signal by smoothing the action signal;

obtaining a plurality of sets of framed signals by framing the envelope signal using a plurality of time windows of different window lengths, respectively;

calculating a periodic expression of each set of framed signals in the plurality of sets of framed signals;

determining a set of transition first target intervals for each set of frame signals based on the periodic expression of each set of frame signals in the plurality of sets of frame signals;

selecting, from a plurality of sets of transition first target intervals, a set of transition first target intervals that satisfies a preference rule as a set of candidate first target intervals; and

generating the first target interval by merging or filtering the set of candidate first target intervals based on a post-processing rule.

13. The method of claim 12, wherein the preference rule includes:

a sum of interval periodic indexes of the set of transition first target intervals being maximum, or

the set of transition first target intervals with a more forward starting point.

14. The method of claim 7, wherein the determining the first target interval at least based on the periodic expression includes:

obtaining a trained machine learning model; and determining the first target interval by inputting the periodic expression and a period of each frame signal into the trained machine learning model.

15. The method of claim 14, wherein the obtaining a trained machine learning model includes:

obtaining a plurality of sample action signals, wherein each of the plurality of sample action signals includes a label of a sample first target interval;

determining a sample periodic expression and a sample period of each sample action signal; and obtaining the trained machine learning model by training an initial machine learning model based on the sample periodic expression and the sample period of each sample action signal and the corresponding label of the sample first target interval.

16. The method of claim 2, wherein the identifying a first target interval in the action signal includes:

obtaining a trained machine learning model; and determining the first target interval by inputting the action signal into the trained machine learning model.

17. The method of claim 16, wherein the obtaining a trained machine learning model includes:

obtaining a plurality of sample action signals, wherein each of the plurality of sample action signals includes a label of a sample first target interval;

obtaining the trained machine learning model by training an initial machine learning model based on each of the plurality of sample action signals and the corresponding label of the sample first target interval.

18. The method of claim 1, wherein the extracting, from the first target interval, a second target interval that is capable of reflecting at least one motion cycle of the target action includes:

determining one or more key points in the first target interval; and
determining the second target interval based on the one or more key points.

19. The method of claim 18, wherein the second target interval corresponds to a force exerting starting point and a force exerting ending point of the at least one motion cycle of the target action, and the determining the second target interval based on the one or more key points includes:
centering on each of the one or more key points, taking a point with a magnitude that is a certain multiple of a maximum value of amplitude as the force exerting starting point and the force exerting ending point of the at least one motion cycle of the target action corresponding to the second target interval.

20. The method of claim 1, wherein the action signal includes signals from a plurality of channels, a signal of each channel corresponding to one type of force exertion muscle, and the identifying a first target interval in the action signal includes:

determining a channel periodic expression relating to the signal of each channel, wherein the channel periodic expression includes periodic indexes of the signal of the corresponding channel;
determining one or more key channels based on a plurality of channel periodic expressions relating to the signals of the plurality of channels; and
determining the first target interval based on channel periodic expressions of the one or more key channels.

21. The method of claim 20, wherein the determining one or more key channels based on a plurality of periodic expressions relating to the signals of the plurality of channels includes:

determining a channel first target interval of each of the plurality of channels based on the plurality of channel periodic expressions; and
determining the one or more key channels based on an amplitude of the channel first target interval of each of the plurality of channels and a value of a periodic index corresponding to the channel first target interval.

22. The method of claim 20, wherein the determining the first target interval based on channel periodic expressions of the one or more key channels includes:

determining, based on the channel periodic expressions, one or more key first target intervals of the one or more key channels, respectively; and
determining the first target interval based on the one or more key first target intervals.

23. The method of claim 22, wherein the determining the first target interval based on the one or more key first target intervals includes:

determining an intersection or a union between times corresponding to the one or more key first target intervals as the first target interval, or determining an intersection or a union between times corresponding to key first target intervals that satisfy a preset condition in the one or more key first target intervals as the first target interval.

24. The method of claim 22, wherein the second target interval corresponds to a force exerting starting point and a force exerting ending point of the at least one motion cycle of the target action, and the extracting, from the first target interval, a second target interval that is capable of reflecting at least one motion cycle of the target action includes:

determining one or more key points in a key first target interval of each of the one or more key channels; and
determining, based on the one or more key points of each of the one or more key channels, the force exerting starting point and the force exerting ending point of the at least one motion cycle of the target action corresponding to the second target interval.

25. The method of claim 24, wherein the determining, based on the one or more key points of each of the one or more key channels, the force exerting starting point and the force exerting ending point of the at least one motion cycle of the target action corresponding to the second target interval includes:

determining, by centering on each of the one or more key points, a sub-force exerting starting point and a sub-force exerting ending point corresponding to the each of the one or more key points to obtain a candidate force segment;
determining a force exertion sequence of force exertion muscles corresponding to the plurality of channels; and
determining the force exerting starting point and the force exerting ending point of the at least one motion cycle of the target action corresponding to the second target interval by combining candidate force segments that satisfy the force exertion sequence.

26. The method of claim 1, wherein the evaluating the target action includes at least one of:

identifying a motion type of the target action, analyzing whether the target action is standard, determining a percentage of muscle consumption,
analyzing muscle activation timing and muscle force exertion, or
analyzing whether the user is fatigued.

27. A system for motion monitoring, comprising:

an acquisition module configured to obtain an action signal of a user;
a first target interval identification module configured to identify a first target interval in the action signal, the first target interval corresponding to a target action of the user;
a second target interval extraction module configured to extract, from the first target interval, a second target interval that is capable of reflecting at least one motion cycle of the target action; and
an evaluation module configured to evaluate the target action according to the second target interval.

28. A device for motion monitoring, comprising at least one processor and at least one memory, wherein:

the at least one memory is configured to store instructions;
the at least one processor is configured to execute the instructions to implement the method for motion monitoring of any one of claims 1-26.

**100**

110

130

130-1

130-2

130-3

130-4

120

Network

140

140-1

140-2

140-3

FIG. 1

**200**

Acquisition module
210

First target interval
identification module
220

Second target interval
extraction module
230

Evaluation module
240

**FIG. 2**

300

Obtaining an action signal of a user ⟋310

Identifying a first target interval in the action signal, the first target interval corresponding to a target action of the user ⟋320

Extracting, from the first target interval, a second target interval that is capable of reflecting at least one motion cycle of the target action ⟋330

Evaluating the target action according to the second target interval ⟋340

**FIG. 3**

400

Obtaining an envelope signal of the action signal by smoothing the action signal ⌐410

↓

Framing the envelope signal using a time window ⌐420

↓

Calculating a periodic index for each frame signal ⌐430

↓

Determining the first target interval based on the periodic expression ⌐440

FIG. 4

**FIG. 5A**

**FIG. 5B**

FIG. 6A

FIG. 6B

700

Obtaining an envelope signal of an action signal by smoothing the action signal ⌐710

Obtaining a plurality of sets of framed signals by framing the envelope signal using a plurality of time windows of different window lengths, respectively ⌐720

Calculating a periodic expression of each set of framed signals in a plurality of sets of framed signals ⌐730

Determining a set of transition first target intervals for the each set of frame signals based on the periodic expression of each set of frame signals in the plurality of sets of frame signals ⌐740

Selecting, from a plurality of sets of transition first target intervals, a set of transition first target intervals that satisfies a preference rule as a set of candidate first target intervals ⌐750

Generating a first target interval by merging or filtering the set of candidate first target intervals based on a post-processing rule ⌐760

**FIG. 7**

**800**

Obtaining an action signal of a user, the action signal including signals from a plurality of channels — 810

Determining a channel periodic expression relating to the signal of each channel, the channel periodic expression including periodic indexes of the signal of the corresponding channel — 820

Determining a channel first target interval of each channel based on the plurality of channel periodic expressions — 830

Determining one or more key channels based on the channel first target interval of each channel — 840

Determining a first target interval based on the one or more key channels — 850

**FIG. 8**

900

FIG. 9

**1000**

```
┌─────────────────────────────────────────────────┐
│                                                 │──1010
│   Determining one or more key points in a key   │
│   first target interval of each key channel     │
│                                                 │
└─────────────────────────────────────────────────┘
                        │
                        ▼
┌─────────────────────────────────────────────────┐
│   Determining, by centering on each of the one  │──1020
│   or more key points, a sub-force exerting      │
│   starting point and a sub-force exerting       │
│   ending point corresponding to each of the     │
│   one or more key points to obtain a            │
│   candidate force segment                       │
└─────────────────────────────────────────────────┘
                        │
                        ▼
┌─────────────────────────────────────────────────┐
│   Determining a force exerting starting point   │──1030
│   and a force exerting ending point of at least │
│   one motion cycle of a target action           │
│   corresponding to a second target interval     │
└─────────────────────────────────────────────────┘
```

**FIG. 10**

FIG. 11

| INTERNATIONAL SEARCH REPORT | International application No. |
| --- | --- |
| | **PCT/CN2022/114990** |

**A.   CLASSIFICATION OF SUBJECT MATTER**

A61B 5/22(2006.01)i

According to International Patent Classification (IPC) or to both national classification and IPC

**B.   FIELDS SEARCHED**

Minimum documentation searched (classification system followed by classification symbols)

IPC:A61B

Documentation searched other than minimum documentation to the extent that such documents are included in the fields searched

Electronic data base consulted during the international search (name of data base and, where practicable, search terms used)

CNTXT, CNKI, ENTXT, DWPI: 运动, 动作, 信号, 监控, 目标, 区间, 周期, 时间, 肌电, 姿态, 曲线, 阈值, 机器学习, 模型, 关键点, 通道, movement, action, signal, monitoring, target, interval, cycle, time, electromyography, attitude, curve, threshold, machine learning, model, key point

**C.   DOCUMENTS CONSIDERED TO BE RELEVANT**

| Category* | Citation of document, with indication, where appropriate, of the relevant passages | Relevant to claim No. |
| --- | --- | --- |
| X | CN 109864741 A (KINGFAR INTERNATIONAL INC.) 11 June 2019 (2019-06-11) description, paragraphs [0007]-[0159] | 1-8, 14-23, 26-28 |
| X | CN 113569606 A (TENCENT TECHNOLOGY (SHENZHEN) CO., LTD.) 29 October 2021 (2021-10-29) description, paragraphs [0005]-[0300] | 1-4, 16-17, 26-28 |
| A | CN 110263870 A (SHENZHEN YUEDONG TIANXIA TECHNOLOGY CO., LTD.) 20 September 2019 (2019-09-20) entire description | 1-28 |
| A | CN 111488824 A (BEIJING BAIDU NETCOM SCIENCE AND TECHNOLOGY CO., LTD.) 04 August 2020 (2020-08-04) entire description | 1-28 |
| A | CN 113749652 A (THE SECOND XIANGYA HOSPITAL OF CENTRAL SOUTH UNIVERSITY) 07 December 2021 (2021-12-07) entire description | 1-28 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **08 March 2023** | **30 March 2023** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

<div align="center">**INTERNATIONAL SEARCH REPORT**</div>

| International application No. |
|---|
| **PCT/CN2022/114990** |

**C. DOCUMENTS CONSIDERED TO BE RELEVANT**

| Category* | Citation of document, with indication, where appropriate, of the relevant passages | Relevant to claim No. |
|---|---|---|
| A | US 2006099556 A1 (SAMSUNG ELECTRONICS CO., LTD.) 11 May 2006 (2006-05-11) entire description | 1-28 |
| A | US 2014303759 A1 (SSTATZZ OY) 09 October 2014 (2014-10-09) entire description | 1-28 |
| A | US 2016035206 A1 (UNIVERSAL SCIENTIFIC INDUSTRIAL (SHANGHAI) CO., LTD.) 04 February 2016 (2016-02-04) entire description | 1-28 |
| A | US 2021362005 A1 (NEC CORP.) 25 November 2021 (2021-11-25) entire description | 1-28 |

Form PCT/ISA/210 (second sheet) (July 2022)

INTERNATIONAL SEARCH REPORT

Information on patent family members

International application No.

**PCT/CN2022/114990**

| Patent document cited in search report | | | Publication date (day/month/year) | Patent family member(s) | | | Publication date (day/month/year) |
|---|---|---|---|---|---|---|---|
| CN | 109864741 | A | 11 June 2019 | None | | | |
| CN | 113569606 | A | 29 October 2021 | HK | 40054511 | A0 | 25 February 2022 |
| CN | 110263870 | A | 20 September 2019 | None | | | |
| CN | 111488824 | A | 04 August 2020 | US | 2021319213 | A1 | 14 October 2021 |
| CN | 113749652 | A | 07 December 2021 | None | | | |
| US | 2006099556 | A1 | 11 May 2006 | KR | 20060040499 | A | 10 May 2006 |
| | | | | KR | 100634523 | B1 | 16 October 2006 |
| US | 2014303759 | A1 | 09 October 2014 | EP | 2793207 | A2 | 22 October 2014 |
| | | | | EP | 2793207 | A3 | 05 November 2014 |
| | | | | US | 9881206 | B2 | 30 January 2018 |
| US | 2016035206 | A1 | 04 February 2016 | TW | 201606711 | A | 16 February 2016 |
| | | | | TWI | 592910 | B | 21 July 2017 |
| | | | | US | 9384646 | B2 | 05 July 2016 |
| | | | | FR | 3024348 | A1 | 05 February 2016 |
| | | | | DE | 102014115223 | A1 | 04 February 2016 |
| | | | | GB | 201418778 | D0 | 03 December 2014 |
| | | | | GB | 2528996 | A | 10 February 2016 |
| | | | | GB | 2528996 | B | 24 August 2016 |
| US | 2021362005 | A1 | 25 November 2021 | WO | 2019150962 | A1 | 08 August 2019 |
| | | | | SG | 10201800954 | QA | 27 September 2019 |
| | | | | JP | 2021511126 | A | 06 May 2021 |
| | | | | JP | 6973653 | B2 | 01 December 2021 |

Form PCT/ISA/210 (patent family annex) (July 2022)